# EUROPÄISCHE PATENTSCHRIFT

(11) **EP 2 683 702 B1**
(45) Veröffentlichungstag und Bekanntmachung des Hinweises auf die Patenterteilung: **24.12.2014**
(21) Anmeldenummer: 12707761.8
(22) Anmeldetag: 07.03.2012
(51) Int. Cl.: C07D 291/06, C07D 419/10, A61K 31/54, A61P 3/10

(54) **NEUE SUBSTITUIERTE PHENYL-OXATHIAZINDERIVATE, VERFAHREN ZU DEREN HERSTELLUNG, DIESE VERBINDUNGEN ENTHALTENDE ARZNEIMITTEL UND DEREN VERWENDUNG**
NEW SUBSTITUTED PHENYL OXATHIAZINE DERIVATIVES, METHOD FOR THEIR MANUFACTURE, MEDICINES CONTAINING THESE COMPOUNDS AND THEIR APPLICATION
NOUVEAUX DÉRIVÉS DE PHÉNYLE-OXATHIAZINE SUBSTITUÉS, LEUR PROCÉDÉ DE FABRICATION, MÉDICAMENT CONTENANT CES LIAISONS ET SON UTILISATION

(30) Priorität: 08.03.2011 EP 11305238
(43) Veröffentlichungstag der Anmeldung: 15.01.2014
(73) Patentinhaber: SANOFI, 75008 Paris (FR)
(72) Erfinder: BOEHME, Thomas, 65926 Frankfurt am Main (DE); RITTER, Kurt, 65926 Frankfurt am Main (DE); ENGEL, Christian, 65926 Frankfurt am Main (DE); GUESSREGEN, Stefan, 65926 Frankfurt am Main (DE); HAACK, Torsten, 65926 Frankfurt am Main (DE); TSCHANK, Georg, 65926 Frankfurt am Main (DE)
(74) Vertreter: Essler, Frank
(86) Internationale Anmeldenummer: PCT/EP2012/053933
(87) Internationale Veröffentlichungsnummer: WO 2012/120050

(56) Entgegenhaltungen:
- JP-A- 60 214 743
- DATABASE CAPLUS [Online] CHEMICAL ABSTRACTS SERVICE, COLUMBUS, OHIO, US; SUZUE, SEIGO ET AL: "Hypoglycemic agents. IV. Synthesis of 1,4,3-benzoxathiazine 4,4-dioxides", XP002656994, gefunden im STN Database accession no. 1968:467344

## Beschreibung

Die Erfindung betrifft substituierte Phenyl-Oxathiazinderivate und deren physiologisch verträgliche Salze.

Der Erfindung lag die Aufgabe zugrunde, Verbindungen zur Verfügung zu stellen, die eine therapeutisch verwertbare Wirkung entfalten. Insbesondere bestand die Aufgabe darin, neue Verbindungen zu finden, die zur Behandlung von Diabetes, Hyperglykämie, Insulin Resistenz, Adipositas oder Lipidstoffwechselstörungen geeignet sind.

Die Erfindung betrifft daher Verbindungen der Formel I 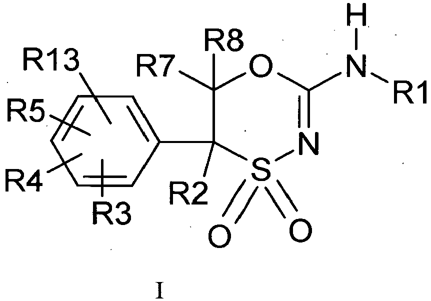 worin bedeuten
- R1: 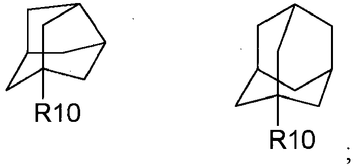
- R2: H, F, (C₁-C₃)-Alkyl wobei der Alkylrest ein bis 3-fach mit Fluor substituiert sein kann;
- R3, R4, R5, R13: unabhängig voneinander
H, F, Cl, Br, I, OH, CF₃, CHF₂, CH₂F, NO₂, CN, OCF₃, OCHF₂, -(C₁-C₆)-Alkylen-(R9), O-(C₁-C₆)-Alkylen-(R9), tertiär-Butyl, iso-Propylen-(R9), (C=O)-(C₁-C₆)-Alkylen-(R9), (C₁-C₆)-Alkylen-(R9), NH₂, NH(C₁-C₆)-Alkylen-(R9), N((C₁-C₆)-Alkylen-R9)₂, (C₁-C₆)-Alkylen-NH₂, (C₁-C₆)-Alkylen-NH(C₁-C₆)-Alkylen-(R9), (C₁-C₆)-Alkylen-N((C₁-C₆)-Alkylen-R9)₂,-O-(C₁-C₆)-Alkylen-NH₂, -O-(C₁-C₆)-Alkylen-NH(C₁-C₆)-Alkylen-(R9), -O-(C₁-C₆)-Alkylen-N((C₁-C₆)-Alkylen-R9)₂, -NH-(C₁-C₆)-Alkylen-NH₂, -NH-(C₁-C₆)-Alkylen-NH(C₁-C₆)-Alkylen-(R9), -NH-(C₁-C₆)-Alkylen-N((C₁-C₆)-Alkylen-R9)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
(C₆-C₁₀)-Aryl, -(C₁-C₆)-Alkylen-(C₆-C₁₀)-Aryl, ,
wobei der Arylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅, (C₆-C₁₀)-Aryl, (C₃-C₇)-Cycloalkyl, 4 bis12-gliedriger Heterocyclus;
wobei der (C₆-C₁₀)-Arylrest, (C₃-C₇)-Cycloalkylrest, 4 bis12-gliedriger Heterocyclusrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
4 bis 12-gliedriger Heterocyclus, -(C₁-C₆)-Alkylen-4 bis 12-gliedriger Heterocyclus,
wobei der Heterocyclylrest ein bis 3-fach substituiert sein kann mit F, Cl, Br, I, OH, CF₃, CHF₂, CH₂F, NO₂, CN, OCF₃, OCHF₂, O-(C₁-C₆)-Alkylen-(R9), (C₁-C₆)-Alkylen-(R9), NH₂, NH(C₁-C₆)-Alkylen-(R9), N((C₁-C₆)-Alkyl)₂, SO₂-(C₁-C₆)-Alkylen-(R9), SO₂-C₂H₂F₃, SO₂-CH₃, SO₂-NH₂, SO₂-NH(C₁-C₆)-Alkylen-(R9), SO₂-N((C₁-C₆)-Alkyl)₂, COOH, COO-(C₁-C₆)-Alkylen-(R9), CONH₂, CONH(C₁-C₆)-Alkylen-(R9), CON((C₁-C₆)-Alkyl)₂, SF₅, (C₆-C₁₀)-Aryl, (C₃-C₇)-Cycloalkyl, 4 bis 12-gliedriger Heterocyclus;
wobei der (C₆-C₁₀)-Arylrest, (C₃-C₇)-Cycloalkylrest, 4 bis12-gliedriger Heterocyclusrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
- R7, R8: unabhängig voneinander H, (C₁-C₃)-Alkyl, wobei der Alkylrest ein bis 3-fach mit Fluor substituiert sein kann, oder R7 und R8 bilden gemeinsam mit dem Kohlenstoffatom an das sie gebunden sind einen 3-7 gliedrigen Carbocyclus oder Heterocyclus;
- R9: H, OH, OCH₃, OCF₃, CHF₂, CF₃;
- R₁₀: H, CONH₂;
sowie deren pharmazeutisch verträgliche Salze.

Eine weitere Ausführungsform betrifft Verbindungen der Formel I, worin ein oder mehrere Reste die folgenden Bedeutungen haben:
- R1: 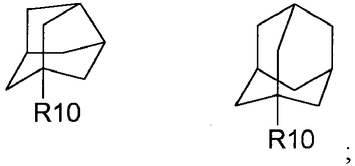
- R2: H, (C₁-C₃)-Alkyl;
- R3, R4, R5, R13: unabhängig voneinander
H, F, Cl, Br, I, OH, CF₃, CHF₂, CH₂F, NO₂, CN, OCF₃, OCHF₂, -(C₁-C₆)-Alkylen-(R9), O-(C₁-C₆)-Alkylen-(R9), tertiär-Butyl, iso-Propylen-(R9), (C=O)-(C₁-C₆)-Alkylen-(R9), (C₁-C₆)-Alkylen-(R9), NH₂, NH(C₁-C₆)-Alkylen-(R9), N((C₁-C₆)-Alkylen-R9)₂, (C₁-C₆)-Alkylen-NH₂, (C₁-C₆)-Alkylen-NH(C₁-C₆)-Alkylen-(R9), (C₁-C₆)-Alkylen-N((C₁-C₆)-Alkylen-R9)₂, -O-(C₁-C₆)-Alkylen-NH₂, -O-(C₁-C₆)-Alkylen-NH(C₁-C₆)-Alkylen-(R9), -O-(C₁-C₆)-Alkylen-N((C₁-C₆)-Alkylen-R9)₂, -NH-(C₁-C₆)-Alkylen-NH₂, -NH-(C₁-C₆)-Alkylen-NH(C₁-C₆)-Alkylen-(R9), -NH-(C₁-C₆)-Alkylen-N((C₁-C₆)-Alkylen-R9)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅,
(C₆-C₁₀)-Aryl, -(C₁-C₆)-Alkylen-(C₆-C₁₀)-Aryl,,
wobei der Arylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N(C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅, (C₆-C₁₀)-Aryl, (C₃-C₇)-Cycloalkyl, 4 bis12-gliedriger Heterocyclus; wobei der (C₆-C₁₀)-Arylrest, (C₃-C₇)-Cycloalkylrest, 4 bis 12-gliedriger Heterocyclusrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
4 bis 12-gliedriger Heterocyclus, -(C₁-C₆)-Alkylen-4 bis12-gliedriger Heterocyclus,
wobei der Heterocyclylrest ein bis 3-fach substituiert sein kann mit F, Cl, Br, I, OH, CF₃, CHF₂, CH₂F, NO₂, CN, OCF₃, OCHF₂, O-(C₁-C₆)-Alkylen-(R9), (C₁-C₆)-Alkylen-(R9), NH₂, NH(C₁-C₆)-Alkylen-(R9), N((C₁-C₆)-Alkyl)₂, SO₂-(C₁-C₆)-Alkylen-(R9), SO₂-C₂H₂F₃, SO₂-CH₃, SO₂-NH₂, SO₂-NH(C₁-C₆)-Alkylen-(R9), SO₂-N((C₁-C₆)-Alkyl)₂, COOH, COO-(C₁-C₆)-Alkylen-(R9), CONH₂, CONH(C₁-C₆)-Alkylen-(R9), CON((C₁-C₆)-Alkyl)₂, SF₅, (C₆-C₁₀)-Aryl, (C₃-C₇)-Cycloalkyl, 4 bis12-gliedriger Heterocyclus; wobei der (C₆-C₁₀)-Arylrest, (C₃-C₇)-Cycloalkylrest, 4 bis12-gliedriger Heterocyclusrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
- R7, R8: unabhängig voneinander H, (C₁-C₃)-Alkyl,
- R9: H, OH, OCH₃, OCF₃, CHF₂, CF₃;
- R₁₀: H, CONH₂;
sowie deren pharmazeutisch verträgliche Salze.

Eine weitere Ausführungsform betrifft Verbindungen der Formel I, worin ein oder mehrere Reste die folgenden Bedeutungen haben:
- R1: 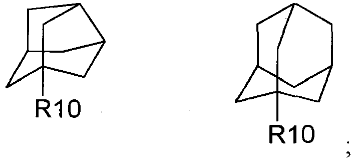
- R2: H;
- R3, R4, R5, R13: unabhängig voneinander H, F, Cl, Br, I, OH, CF₃, NO₂, CN, OCF₃, OCHF₂, -(C₁-C₆)-Alkylen, -O-(C₁-C₆)-Alkylen;
(C₆-C₁₀)-Aryl, wobei der Arylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅; 4 bis 12-gliedriger Heterocyclus, wobei der Heterocyclylrest ein bis 3-fach substituiert sein kann mit F, Cl, Br, I, OH, CF₃, CHF₂, CH₂F, NO₂, CN, OCF₃, OCHF₂, O-(C₁-C₆)-Alkylen-(R9), (C₁-C₆)-Alkylen-(R9), NH₂, NH(C₁-C₆)-Alkylen-(R9), N((C₁-C₆)-Alkyl)₂, SO₂-(C₁-C₆)-Alkylen-(R9), SO₂-C₂H₂F₃, SO₂-CH₃, SO₂-NH₂, SO₂-NH(C₁-C₆)-Alkylen-(R9), SO₂-N((C₁-C₆)-Alkyl)₂, COOH, COO-(C₁-C₆)-Alkylen-(R9), CONH₂, CONH(C₁-C₆)-Alkylen-(R9), CON((C₁-C₆)-Alkyl)₂, SF₅;
- R7, R8: H, (C₁-C₃)-Alkyl;
- R9: H, OH, OCH₃, OCF₃, CHF₂, CF₃;
- R₁₀: H, CONH₂;
sowie deren pharmazeutisch verträgliche Salze.

Eine weitere Ausführungsform betrifft Verbindungen der Formel I, worin ein oder mehrere Reste die folgenden Bedeutungen haben:
- R1: 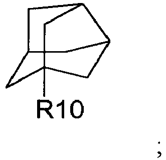
- R2: H;
- R3, R4, R5: unabhängig voneinander
H, F, Cl, Br, I, OH, CF₃, NO₂, CN, OCF₃, OCHF₂, -(C₁-C₆)-Alkylen, -O-(C₁-C₆)-Alkylen;
Phenyl,
wobei der Phenylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(Ci-C₆)-Alkyl, CONH₂, CONH(C₁-C₆)-Alkyl, CON((C₁-C₆)-Alkyl)₂, SF₅,
Piperidinyl,
wobei der Piperidinyllrest ein bis 3-fach substituiert sein kann mit F, Cl, Br, I, OH, CF₃, CHF₂, CH₂F, NO₂, CN, OCF₃, OCHF₂, O-(C₁-C₆)-Alkylen-(R9), (C₁-C₆)-Alkylen-(R9), NH₂, NH(C₁-C₆)-Alkylen-(R9), N((C₁-C₆)-Alkyl)₂, SO₂-(C₁-C₆)-Alkylen-(R9), SO₂-C₂H₂F₃, SO₂-CH₃, SO₂-NH₂, SO₂-NH(C₁-C₆)-Alkylen-(R9), SO₂-N((C₁-C₆)-Alkyl)₂, COOH, COO-(C₁-C₆)-Alkylen-(R9), CONH₂, CONH(C₁-C₆)-Alkylen-(R9), CON((C₁-C₆)-Alkyl)₂, SF₅;
- R13: F, Cl, Br, I, OH, CF₃, NO₂, CN, OCF₃, OCHF₂, -(C₁-C₆)-Alkylen, -O-(C₁-C₆)-Alkylen;
Phenyl,
wobei der Phenylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
Piperidinyl,
wobei der Piperidinyllrest ein bis 3-fach substituiert sein kann mit F, Cl, Br, I, OH, CF₃, CHF₂, CH₂F, NO₂, CN, OCF₃, OCHF₂, O-(C₁-C₆)-Alkylen-(R9), (C₁-C₆)-Alkylen-(R9), NH₂, NH(C₁-C₆)-Alkylen-(R9), N((C₁-C₆)-Alkyl)₂, SO₂-(C₁-C₆)-Alkylen-(R9), SO₂-C₂H₂F₃, SO₂-CH₃, SO₂-NH₂, SO₂-NH(C₁-C₆)-Alkylen-(R9), SO₂-N((C₁-C₆)-Alkyl)₂, COOH, COO-(C₁-C₆)-Alkylen-(R9), CONH₂, CONH(C₁-C₆)-Alkylen-(R9), CON((C₁-C₆)-Alkyl)₂, SF₅;
- R7, R8: H, (C₁-C₃)-Alkyl;
- R9: H, OH, OCH₃, OCF₃, CHF₂, CF₃;
- R₁₀: H, CONH₂;
sowie deren pharmazeutisch verträgliche Salze.

Eine weitere Ausführungsform betrifft Verbindungen der Formel I, worin der Rest R1 wie in Formel Ia und Ib dargestellt verknüpft ist.

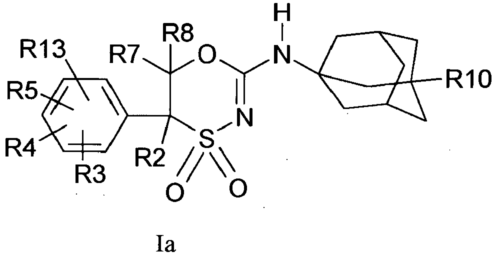

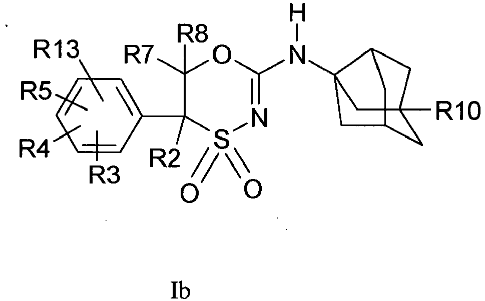

Können Reste oder Substituenten mehrfach in den Verbindungen der Formeln I auftreten, so können sie alle unabhängig voneinander die angegebene Bedeutung haben und gleich oder verschieden sein.

Die Erfindung bezieht sich auf Verbindungen der Formel I, in Form ihrer Tautomere, Racemate, racemischen Mischungen, Stereoisomerengemische, reinen Stereoisomere, Diastereoisomerengemische, reinen Diastereoisomere. Die Trennung der Gemische erfolgt zum Beispiel auf chromatographischem Weg.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Bernstein-, p-Toluolsulfon- und Weinsäure. Geeignete pharmazeutisch verträgliche basische Salze sind Ammoniumsalze, Alkalimetallsalze (wie Natrium- und Kaliumsalze), Erdalkalisalze (wie Magnesium- und Calciumsalze), Trometamol (2-Amino-2-hydroxymethyl-1,3-propandiol), Diethanolamin, Lysin oder Ethylendiamin.

Salze mit einem nicht pharmazeutisch verträglichen Anion, wie zum Beispiel Trifluoracetat, gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel I" auf Verbindung(en) der Formel I wie vorstehend beschrieben, sowie ihre Salze und Solvate wie hierin beschrieben.

Unter einem Alkylrest wird eine geradkettige oder verzweigte Kohlenwasserstoffkette mit einem bis acht Kohlenstoffen verstanden, wie z.B. Methyl, Ethyl, iso-Propyl, tert.-Butyl, Hexyl, Heptyl, Octyl. Die Alkylreste können einfach oder mehrfach wie oben beschrieben substituiert sein.

Unter einem Alkylenrest wird eine geradkettige oder verzweigte Kohlenwasserstoffkette mit zwei freien Valenzen verstanden, wie z.B. Methylen, Ethylen, iso-Propylen, tert.-Butylen.

Unter einem Arylrest wird ein Phenyl, Naphthyl-, Biphenyl-, Tetrahydronaphthyl-, alpha- oder beta-Tetralon-, Indanyl- oder Indan-1-on-ylrest verstanden.

Die Arylreste können ein oder mehrfach mit geeigneten Gruppen wie oben beschrieben substituiert sein.

Unter Heterocyclus bzw. heterocyclischer Rest werden Ringe und Ringsysteme verstanden, die außer Kohlenstoff noch Heteroatome, wie zum Beispiel Stickstoff, Sauerstoff oder Schwefel enthalten. Ferner gehören auch Ringsysteme zu dieser Definition, worin der Heterocylus bzw. der heterocyclische Rest mit einem weiteren Ringsystem anelliert ist. Der Heterocylus bzw. der heterocyclische Rest kann gesättigt, teilweise gesättigt oder aromatisch sein.

Geeignete "Heterocyclen" bzw. "heterocyclische Reste" sind Acridinyl, Azepanyl, Azocinyl, Benzimidazolyl, Benzofuryl, Benzothienyl, Benzothiophenyl, Benzoxazolyl, Benzthiazolyl, Benztriazolyl, Benztetrazolyl, Benzisoxazolyl, Benzisothiazolyl, Benzimidazalinyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Decahydrochinolinyl, 2H,6H-1,5,2-Dithiazinyl, Dihydrofuro[2,3-b]-Tetrahydrofuran, 5,6-Dihydro-4H-cyclopentathiazol-2-yl, 4,5-Dihydro-thiazol-2-yl, Furyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl (Benzimidazolyl), Isothiazolyl, Isoxazolyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxazolidinyl, Pyrimidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purynyl, Pyranyl, Pyrazinyl, Pyroazolidinyl, Pyrazolinyl, Pyrazolyl, Pyridazinyl, Pyridooxazole, Pyridoimidazole, Pyridothiazole, Pyridinyl, Pyridyl, Pyrimidinyl, Pyrrolidinyl, Pyrrolinyl, 2H-Pyrrolyl, Pyrrolyl, 4,5,6,7-Tetrahydro-benzooxazol-2-yl, 4,5,6,7-Tetrahydro-benzothiazol-2-yl, 4,5,6,7-Tetrahydro-benzoimidazol-2-yl, 4,5,6,7-Tetrahydro-pyrazolo[1,5-a]pyridin-2-yl, Tetrahydrofuranyl, Tetrahydropyranyl, Tetrahydroisochinolinyl, Tetrahydrochinolinyl, 6H-1,2,5-Thiadazinyl, Thiazolyl, 1,2,3-Thiadiazolyl, 1,2,4-Thiadiazolyl, 1,2,5-Thiadiazolyl, 1,3,4-Thiadiazolyl, Thienyl, Triazinyl, Triazolyl, Tetrazolyl, Thiazolo[4,5-b]pyridinyl, Thieno[2,3-d]thiazol-2-yl, Tropanyl und Xanthenyl.

Die Heterocyclen bzw. heterocyclischen Reste können ein oder mehrfach mit geeigneten Gruppen wie oben beschrieben substituiert sein.,

Die Verbindung(en) der Formel I können auch in Kombination mit weiteren Wirkstoffen verabreicht werden.

Die Menge einer Verbindung gemäß Formel I, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im Allgemeinen liegt die Tagesdosis im Bereich von 0.3 mg bis 100 mg (typischerweise von 3 mg und 50 mg) pro Tag pro Kilogramm Körpergewicht, z.B. 3-10 mg / kg / Tag. Eine intravenöse Dosis kann z.B. im Bereich von 0.3 mg bis 1.0 mg / kg liegen, die geeigneterweise als Infusion von 10 ng bis 100 ng pro Kilogramm pro Minute verabreicht werden kann. Geeignete Infusionslösungen für diese Zwecke können z.B. von 0.1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1.0 bis 1000 mg, typischerweise von 10 bis 600 mg enthalten. Zur Therapie der oben genannten Zustände können die Verbindungen gemäß Formel I selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0.05 % bis 95 Gew.-% des Wirkstoffs enthalten kann. Weitere pharmazeutisch aktive Substanzen können ebenfalls vorhanden sein, einschließlich weiterer Verbindungen gemäß Formel I. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Poylvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel I enthalten; als Pulver oder Granulate; als Lösung oder Suspension in einer wässrigen oder nicht-wässrigen Flüssigkeit; oder als eine Öl-in-Wasser- oder Wasser-in-Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel I mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen einer Verbindung gemäß Formel I, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0.1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel I mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglykole, Alkohole und Kombinationen von zwei oder mehreren dieser Substanzen verwendet werden. Der Wirkstoff ist im Allgemeinen in einer Konzentration von 0.1 bis 15 Gew.-% der Zusammensetzung vorhanden, beispielsweise von 0.5 bis 2 %.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff-Konzentration beträgt ca. 1 % bis 35 %, vorzugsweise ca. 3 % bis 15 %. Als eine besondere Möglichkeit kann der Wirkstoff, wie beispielsweise in Pharmaceutical Research, 2(6): 318 (1986) beschrieben, durch Elektrotransport oder Iontophorese freigesetzt werden.

Als weitere Wirkstoffe für die Kombinationspräparate sind geeignet:

Alle Antidiabetika, die in der Roten Liste 2006, Kapitel 12 genannt sind; alle Abmagerungsmittel/Appetitzügler, die in der Roten Liste 2006, Kapitel 1 genannt sind; alle Lipidsenker, die in der Roten Liste 2006, Kapitel 58 genannt sind. Sie können mit der erfindungsgemäßen Verbindung der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Die meisten der nachfolgend aufgeführten Wirkstoffe sind in USP Dictionary ofUSAN and International Drug Names, US Pharmacopeia, Rockville 2001, offenbart.

Antidiabetika umfassen Insulin und Insulinderivate, wie z.B. Lantus^{®} (siehe www.lantus.com) oder HMR 1964 oder Levemir^{®} (insulin detemir) oder solche, wie sie in WO2005005477 (Novo Nordisk) beschrieben sind, schnell wirkende Insuline (siehe US 6,221,633), inhalierbare Insuline, wie z. B. Exubera^{®} oder orale Insuline, wie z. B. IN-105 (Nobex) oder Oral-lyn ™ (Generex Biotechnology), GLP-1-Derivate wie z.B. Exenatide, Liraglutide oder diejenigen die in WO 98/08871, WO2005027978, WO2006037811, WO2006037810 von Novo Nordisk A/S, in WO 01/04156 von Zealand oder in WO 00/34331 von Beaufour-Ipsen offenbart wurden, Pramlintide Acetat (Symlin; Amylin Pharmaceuticals), sowie oral wirksame hypoglykämische Wirkstoffe.

Die oral wirksamen hypoglykämischen Wirkstoffe umfassen vorzugsweise Sulfonylharnstoffe, Biguanidine,
Meglitinide,
Oxadiazolidindione,
Thiazolidindione,
Glukosidase-Inhibitoren,
Hemmstoffe der Glykogenphosphorylase,
Glukagon-Antagoni sten,
Glukokinaseaktivatoren,
Inhibitoren der Fructose-1,6-bisphosphatase
Modulatoren des Glukosetransporters-4 (GLUT4),
Inhibitoren der Glutamin-Fructose-6-Phosphat-Amidotransferase (GFAT),
GLP-1-Agonisten, Kaliumkanalöffner, wie z.B. diejenigen, die in WO 97/26265 und WO 99/03861 von Novo Nordisk A/S offenbart wurden oder die, die in WO2006045799 beschrieben sind (Solvay),
Inhibitoren der Dipeptidylpeptidase-IV (DPP-IV),
Insulin-Sensitizer,
Inhibitoren von Leberenzymen, die an der Stimulation der Glukoneogenese und/oder Glykogenolyse beteiligt sind,
Modulatoren der Glukoseaufnahme, des Glukosetransports und der Glukoserückresorption, Hemmstoffe der 11β-HSD1,
Inhibitoren der Protein-Tyrosin-Phosphatase-1B (PTP1B),
Modulatoren des natrium-abhängigen Glukosetransporters 1 oder 2 (SGLT1, SGLT2),
den Fettstoffwechsel verändernde Verbindungen wie antihyperlipidämische Wirkstoffe und antilipidämische Wirkstoffe,
Verbindungen, die die Nahrungsmitteleinnahme verringern,
Verbindungen, die die Thermogenese erhöhen,
PPAR- und RXR-Modulatoren und
Wirkstoffe, die auf den ATP-abhängigen Kaliumkanal der Betazellen wirken.

Bei einer Ausführungsform der Erfindung wird die Verbindungen der Formel I in Kombination mit einem HMGCoA-Reduktase Inhibitor wie Simvastatin, Fluvastatin, Pravastatin, Lovastatin, Atorvastatin, Cerivastatin, Rosuvastatin, L-659699 verabreicht.

Bei einer Ausführungsform der Erfindung wird die Verbindung der Formel I in Kombination mit einem Cholesterinresorptionsinhibitor, wie z.B. Ezetimibe, Tiqueside, Pamaqueside, FM-VP4 (sitostanol/campesterol ascorbyl phosphat; Forbes Medi-Tech, WO2005042692, WO2005005453), MD-0727 (Microbia Inc., WO2005021497, WO2005021495) oder mit Verbindungen, wie in WO2002066464 (Kotobuki Pharmaceutical Co. Ltd.) oder WO2005044256 oder WO2005062824 (Merck & Co.) oder WO2005061451 und WO2005061452 (AstraZeneca AB) und WO2006017257 (Phenomix) oder WO2005033100 (Lipideon Biotechnology AG) beschrieben, verabreicht.

Bei einer Ausführungsform der Erfindung wird die Verbindung der Formel I in Kombination mit Vytorin™, einer festen Kombination von Ezetimibe mit Simvastatin, verabreicht.

Bei einer Ausführungsform der Erfindung wird die Verbindung der Formel I in Kombination mit einer festen Kombination von Ezetimibe mit Fenofibrat verabreicht.

Bei einer weiteren Ausführungsform der Erfindung wird die Verbindung der Formel I in Kombination mit einer festen Kombination von Fenofibrat mit Rosuvastatin verabreicht.

Bei einer Ausführungsform der Erfindung wird die Verbindung der Formel I in Kombination mit ISIS-301012, einem Antisense-Oligonukleotid, welches in der Lage ist, das Apolipoprotein B Gen zu regulieren, verabreicht.

Bei einer Ausführungsform der Erfindung wird die Verbindung der Formel I in Kombination mit einem PPAR gamma Agonisten, wie z.B. Rosiglitazon, Pioglitazon, JTT-501, Gl 262570, R-483, CS-011 (Rivoglitazon) verabreicht.

Bei einer Ausführungsform der Erfindung wird die Verbindung der Formel I in Kombination mit Competact™, einer festen Kombination von Pioglitazon Hydrochlorid mit Metformin Hydrochlorid, verabreicht.

Bei einer Ausführungsform der Erfindung wird die Verbindung der Formel I in Kombination mit duetact™, einer festen Kombination von Pioglitazon Hydrochlorid mit Glimepirid, verabreicht.

Bei einer Ausführungsform der Erfindung wird die Verbindung der Formel I in Kombination mit Avandamet^{®}, einer festen Kombination von Rosiglitazon Maleat mit Metformin Hydrochlorid, verabreicht.

Bei einer Ausführungsform der Erfindung wird die Verbindung der Formel I in Kombination mit PPAR alpha Agonisten, wie z.B. GW9578, GW-590735, K-111, LY-674, KRP-101, DRF-10945 verabreicht.

Bei einer Ausführungsform der Erfindung wird die Verbindung der Formel I in Kombination mit einem gemischten PPAR alpha/gamma Agonisten, wie z.B. Naveglitazar, LY-510929, ONO-5129, E-3030, AVE 8042, AVE 8134, AVE 0847, oder wie in PCT/US 00/11833, PCT/US 00/11490, DE10142734.4 oder in J.P.Berger et al., TRENDS in Pharmacological Sciences 28(5), 244-251, 2005 beschrieben, verabreicht.

Bei einer Ausführungsform der Erfindung wird die Verbindung der Formel I in Kombination mit einem PPAR delta Agonisten, wie z.B. GW-501516 verabreicht.

Bei einer Ausführungsform wird die Verbindung der Formel I in Kombination mit Metaglidasen oder mit MBX-2044 oder anderen partiellen PPAR gamma Agonisten/Antagonisten verabreicht

Bei einer weiteren Ausführungsform der Erfindung wird die Verbindung der Formel I in Kombination mit einem Aktivator der AMP-aktivierten Proteinkinase (AMPK), wie z. B. A-769662 oder solchen Verbindungen wie sie in US20050038068 beschrieben sind, verabreicht.

Bei einer Ausführungsform der Erfindung wird die Verbindung der Formel I in Kombination mit einem Fibrat, wie z.B. Fenofibrat, Clofibrat, Bezafibrat, verabreicht.

Bei einer Ausführungsform der Erfindung wird die Verbindung der Formel I in Kombination mit einem MTP-Inhibitor, wie z.B. Implitapide , BMS-201038, R-103757 oder solchen wie in WO2005085226, WO2005121091, WO2006010423 beschrieben, verabreicht.

Bei einer Ausführungsform der Erfindung wird die Verbindung der Formel I in Kombination mit einem CETP-Inhibitor, wie z.B. Torcetrapib oder JTT-705 oder solchen wie sie in WO2006002342, WO2006010422, WO2006012093 beschrieben sind, verabreicht.

Bei einer Ausführungsform der Erfindung wird die Verbindung der Formel I in Kombination mit Gallensäureresorptionsinhibitor (siehe z.B. US 6,245,744, US 6,221,897 oder WO00/61568), wie z.B. HMR 1741 oder solchen wie in DE 10 2005 033099.1 und DE 10 2005 033100.9 beschrieben, verabreicht.

Bei einer Ausführungsform der Erfindung wird die Verbindung der Formel I in Kombination mit einem polymeren Gallensäureadsorber, wie z.B. Cholestyramin, Colesevelam, verabreicht.

Bei einer Ausführungsform der Erfindung wird die Verbindung der Formel I in Kombination mit einem LDL-Rezeptorinducer (siehe US 6,342,512), wie z.B. HMR1171, HMR1586, oder solchen wie in WO2005097738 beschrieben, verabreicht.

Bei einer Ausführungsform wird die Verbindung der Formel I in Kombination mit Omacor® (Omega-3-Fettsäuren; hochkonzentrierte Ethylester der Eicosapentaensäure und der Docosahexaensäure) verabreicht.

Bei einer Ausführungsform der Erfindung wird die Verbindung der Formel I in Kombination mit einem ACAT-Inhibitor, wie z.B. Avasimibe oder SMP-797, verabreicht.

Bei einer Ausführungsform der Erfindung wird die Verbindung der Formel I in Kombination mit einem Antioxidans, wie z.B. OPC-14117, Probucol, Tocopherol, Ascorbinsäure, β-Caroten oder Selen verabreicht.

Bei einer Ausführungsform der Erfindung wird die Verbindung der Formel I in Kombination mit einem Vitamin, wie z. B. Vitamin B6 oder Vitamin B 12 verabreicht.

Bei einer Ausführungsform der Erfindung wird die Verbindung der Formel I in Kombination mit einem Lipoprotein-Lipase Modulator, wie z.B. Ibrolipim (NO-1886), verabreicht.

Bei einer Ausführungsform der Erfindung wird die Verbindung der Formel I in Kombination mit einem ATP-Citrat-Lyase Inhibitor, wie z.B. SB-204990, verabreicht.

Bei einer Ausführungsform der Erfindung wird die Verbindung der Formel I in Kombination mit einem Squalen Synthetase Inhibitor, wie z.B. BMS-188494 oder wie in WO2005077907 beschrieben, verabreicht.

Bei einer Ausführungsform der Erfindung wird die Verbindung der Formel I in Kombination mit einem Lipoprotein(a) antagonist, wie z.B. Gemcabene (CI-1027) verabreicht.

Bei einer Ausführungsform der Erfindung wird die Verbindung der Formel I in Kombination mit einem Aqonisten des GPR109A (HM74A Rezeptor Agonisten), wie z.B. Nicotinsäure oder "extended release niacin" in Verbindung mit MK-0524A oder solchen Verbindungen, wie sie in WO2006045565, WO2006045564, WO2006069242 beschrieben sind, verabreicht.

Bei einer anderen Ausführungsform der Erfindung wird die Verbindung der Formel I in Kombination mit einem Aqonisten des GPR116, wie sie z.B. in WO2006067531, WO2006067532 beschrieben sind, verabreicht.

Bei einer Ausführungsform der Erfindung wird die Verbindung der Formel I in Kombination mit einem Lipase Inhibitor, wie z.B. Orlistat oder Cetilistat (ATL-962), verabreicht.

Bei einer Ausführungsform der Erfindung wird die Verbindung der Formel I in Kombination mit Insulin verabreicht.

Bei einer Ausführungsform wird die Verbindung der Formel I in Kombination mit einem Sulfonylharnstoff, wie z.B. Tolbutamid, Glibenclamid, Glipizid oder Glimepirid verabreicht.

Bei einer Ausführungsform wird die Verbindung der Formel I in Kombination mit einer die Insulinsekretion verstärkende Substanz, wie z. B. KCP-265 (WO2003097064), verabreicht.

Bei einer Ausführungsform wird die Verbindung der Formel I in Kombination mit Agonisten des glucose-abhängigen insulinotropischen Rezeptors (GDIR) wie z. B. APD-668 verabreicht

Bei einer Ausführungsform wird die Verbindung der Formel I in Kombination mit einem Biguanid, wie z.B. Metformin, verabreicht.

Bei wieder einer anderen Ausführungsform wird die Verbindung der Formel I in Kombination mit einem Meglitinid, wie z.B. Repaglinide oder Nateglinid, verabreicht.

Bei einer Ausführungsform wird die Verbindung der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]phenyl]methyl]-2,4-thiazolidindion, verabreicht.

Bei einer Ausführungsform wird die Verbindung der Formel I in Kombination mit einem α-Glukosidase-Inhibitor, wie z.B. Miglitol oder Acarbose, verabreicht.

Bei einer Ausführungsform wird die Verbindung der Formel I in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid.

Bei einer Ausführungsform wird die Verbindung der Formel I in Kombination mit mehr als einer der vorstehend genannten Verbindungen, z.B. in Kombination mit einem Sulfonylharnstoff und Metformin, einem Sulfonylharnstoff und Acarbose, Repaglinid und Metformin, Insulin und einem Sulfonylharnstoff, Insulin und Metformin, Insulin und Troglitazon, Insulin und Lovastatin, etc. verabreicht.

Bei einer Ausführungsform wird die Verbindung der Formel I in Kombination mit einem Hemmstoff der Glykogenphosphorylase, wie z.B. PSN-357 oder FR-258900 oder solchen wie in WO2003084922, WO2004007455, WO2005073229-31, WO2005067932 beschrieben, verabreicht.

Bei einer Ausführungsform wird die Verbindung der Formel I in Kombination mit Glukagon-Rezeptor-Antagonisten, wie z.B. A-770077 oder NNC-25-2504 oder wie in WO2004100875, WO2005065680, beschrieben, verabreicht.

Bei einer Ausführungsform wird die Verbindung der Formel I in Kombination mit Aktivatoren der Glukokinase, wie z. B. LY-2121260 (WO2004063179), PSN-105, PSN-110, GKA-50 oder solchen wie sie z. B. in WO2004072031, WO2004072066, WO2005080360, WO2005044801, WO2006016194, WO2006058923 beschrieben sind, verabreicht.

Bei einer Ausführungsform wird die Verbindung der Formel I in Kombination mit einem Inhibitor der Glukoneogenese, wie z. B. FR-225654, verabreicht.

Bei einer Ausführungsform wird die Verbindung der Formel I in Kombination mit Inhibitoren der Fructose-1,6-bisphosphatase (FBPase) wie z.B. CS-917 (MB-06322) oder MB-07803 oder solchen wie sie in WO2006023515 beschrieben sind, verabreicht.

Bei einer Ausführungsform wird die Verbindung der Formel I in Kombination mit Modulatoren des Glukosetransporters-4 (GLUT4), wie z. B. KST-48 (D.-O. Lee et al.: Arzneim.-Forsch. Drug Res. 54 (12), 835 (2004)), verabreicht.

Bei einer Ausführungsform wird die Verbindung der Formel I in Kombination mit Inhibitoren der Glutamin-Fructose-6-Phosphat-Amidotransferase (GFAT), wie sie z. B. in WO2004101528 beschrieben sind, verabreicht.

Bei einer Ausführungsform wird die Verbindung der Formel I in Kombination mit Inhibitoren der Dipeptidylpeptidase-IV (DPP-IV), wie z. B. Vildagliptin (LAF-237), Sitagliptin (MK-0431), Saxagliptin ((BMS-477118), GSK-823093, PSN-9301, SYR-322, SYR-619, TA-6666, TS-021, GRC-8200, GW-825964X, KRP-104, DP-893 oder wie sie in WO2003074500, WO2003106456, WO200450658, WO2005058901, WO2005012312, WO2005/012308, WO2006039325, WO2006058064, PCT/EP2005/007821, PCT/EP2005/008005, PCT/EP2005/008002, PCT/EP2005/008004, PCT/EP2005/008283, DE 10 2005 012874.2 oder DE 10 2005 012873.4 beschrieben sind, verabreicht.

Bei einer Ausführungsform wird die Verbindung der Formel I in Kombination mit Januvia™, einer festen Kombination von Sitagliptin Phosphat mit Metformin hydrochlorid, verabreicht.

Bei einer Ausführungsform wird die Verbindung der Formel I in Kombination mit Hemmstoffen der 11-beta-Hydroxysteroid-Dehydrogenase-1 (11β-HSD1), wie z. B. BVT-2733, JNJ-25918646, INCB-13739 oder solche, wie sie z. B. in WO200190090-94, WO200343999, WO2004112782, WO200344000, WO200344009, WO2004112779, WO2004113310, WO2004103980, WO2004112784, WO2003065983, WO2003104207, WO2003104208, WO2004106294, WO2004011410, WO2004033427, WO2004041264, WO2004037251, WO2004056744, WO2004058730, WO2004065351, WO2004089367, WO2004089380, WO2004089470-71, WO2004089896, WO2005016877, WO2005097759, WO2006010546, WO2006012227, WO2006012173, WO2006017542, WO2006034804, WO2006040329, WO2006051662, WO2006048750, WO2006049952, WO2006048331, WO2006050908, WO2006024627, WO2006040329, WO2006066109 beschrieben sind, verabreicht.

Bei einer Ausführungsform wird die Verbindung der Formel I in Kombination mit Inhibitoren der Protein-Tyrosin-Phosphatase-1B (PTP1B), wie sie z. B. in WO200119830-31, WO200117516, WO2004506446, WO2005012295, WO2005116003, PCT/EP2005/005311, PCT/EP2005/005321, PCT/EP2005/007151, PCT/EP2005/01294 oder DE 10 2004 060542.4 beschrieben sind, verabreicht.

Bei einer Ausführungsform wird die Verbindung der Formel I in Kombination mit Modulatoren des natrium-abhängigen Glukosetransporters 1 oder 2 (SGLT1, SGLT2), wie z.B. KGA-2727, T-1095, SGL-0010, AVE 2268 und SAR 7226 oder wie sie z. B. in WO2004007517, WO200452903, WO200452902, PCT/EP2005/005959, WO2005085237, JP2004359630, WO2005121161, WO2006018150, WO2006035796, WO2006062224, WO2006058597 oder von A. L. Handlon in Expert Opin. Ther. Patents (2005) 15(11), 1531-1540 beschrieben sind, verabreicht.

Bei einer Ausführungsform wird die Verbindung der Formel I in Kombination mit Modulatoren des GPR40 verabreicht.

Bei einer Ausführungsform wird die Verbindung der Formel I in Kombination mit Modulatoren des GPR119b, wie sie z. B. in WO2004041274 beschrieben sind, verabreicht.

Bei einer Ausführungsform wird die Verbindung der Formel I in Kombination mit Modulatoren des GPR119, wie sie z. B. in WO2005061489 (PSN-632408) beschrieben sind, verabreicht.

Bei einer Ausführungsform wird die Verbindung der Formel I in Kombination mit Inhibitoren der hormon-sensitiven Lipase (HSL), wie z. B. in WO2005073199 beschrieben, verabreicht.

Bei einer Ausführungsform wird die Verbindung der Formel I in Kombination mit Hemmstoffen der Acetyl-CoA Carboxylase (ACC) wie z. B. solchen wie in WO199946262, WO200372197, WO2003072197, WO2005044814, WO2005108370, JP2006131559 beschrieben, verabreicht.

Bei einer Ausführungsform wird die Verbindung der Formel I in Kombination mit einem Inhibitor der Phosphoenolpyruvatcarboxykinase (PEPCK), wie z.B. solchen, wie in WO2004074288 beschrieben, verabreicht.

Bei einer Ausführungsform wird die Verbindung der Formel I in Kombination mit einem Inhibitor der Glykogen Synthase Kinase-3 beta (GSK-3 beta), wie z. B. in US2005222220, WO2005085230, PCT/EP2005/005346, WO2003078403, WO2004022544, WO2003106410, WO2005058908, US2005038023, WO2005009997, US2005026984, WO2005000836, WO2004106343, EP1460075, WO2004014910, WO2003076442, WO2005087727, WO2004046117 beschrieben.

Bei einer Ausführungsform wird die Verbindung der Formel I in Kombination mit einem Inhibitor der Protein Kinase C beta (PKC beta), wie z. B. Ruboxistaurin, verabreicht.

Bei einer Ausführungsform wird die Verbindung der Formel I in Kombination mit einem Endothelin-A-Rezeptor Antagonisten, wie z. B. Avosentan (SPP-301), verabreicht.

Bei einer Ausführungsform wird die Verbindung der Formel I in Kombination mit Inhibitoren der "I-kappaB kinase" (IKK Inhibitoren), wie sie z. B. in WO2001000610, WO2001030774, WO2004022553, WO2005097129 beschrieben sind, verabreicht.

Bei einer Ausführungsform wird die Verbindung der Formel I in Kombination mit Modulatoren des Glucocorticoidrezeptors, wie sie z. B. in WO2005090336 beschrieben sind, verabreicht.

Bei einer weiteren Ausführungsform wird die Verbindung der Formel I in Kombination mit CART-Modulatoren (siehe "Cocaine-amphetamine-regulated transcript influences energy metabolism, anxiety and gastric emptying in mice" Asakawa, A. et al.: Hormone and Metabolic Research (2001), 33(9), 554-558);
NPY-Antagonisten wie z.B. Naphthalin-1-sulfonsäure-{4-[(4-amino-quinazolin-2-ylamino)-methyl]-cyclohexylmethyl}-amid Hydrochlorid (CGP 71683A);
NPY-5 Rezeptorantagonisten wie L-152804, S-2367 oder wie sie z. B. in WO2006001318 beschrieben sind;
Peptid YY 3-36 (PYY3-36) oder analoge Verbindungen wie z. B. CJC-1682 (PYY3-36 konjugiert mit humanem Serum Albumin über Cys34) oder CJC-1643 (Derivat des PYY3-36, welches sich in vivo an Serum Albumin konjugiert) oder solche, wie sie in WO2005080424 beschrieben sind;
CB1R (Cannabinoid Rezeptor 1) Antagonisten (wie z.B. Rimonabant, SR147778, SLV-319, AVE-1625, MK-0364 oder Salze davon oder solche wie sie in z. B. EP 0656354, WO 00/15609, WO2001/64632, WO2001/64633, WO2001/64634, WO 02/076949, WO2005080345, WO2005080328, WO2005080343, WO2005075450, WO2005080357, WO200170700, WO2003026647-48, WO200302776, WO2003040107, WO2003007887, WO2003027069, US6,509,367, WO200132663, WO2003086288, WO2003087037, WO2004048317, WO2004058145, WO2003084930, WO2003084943, WO2004058744, WO2004013120, WO2004029204, WO2004035566, WO2004058249, WO2004058255, WO2004058727, WO2004069838, US20040214837, US20040214855, US20040214856, WO2004096209, WO2004096763, WO2004096794, WO2005000809, WO2004099157, US20040266845, WO2004110453, WO2004108728, WO2004000817, WO2005000820, US20050009870, WO200500974, WO2004111033-34, WO200411038-39, WO2005016286, WO2005007111, WO2005007628, US20050054679, WO2005027837, WO2005028456, WO2005063761-62, WO2005061509, WO2005077897, WO2006047516, WO2006060461, WO2006067428, WO2006067443 beschrieben sind);
MC4-Agonisten (z.B. 1-Amino-1,2,3,4-tetrahydro-naphthalin-2-carbonsäure [2-(3a-benzyl-2-methyl-3-oxo-2,3,3a,4,6,7-hexahydro-pyrazolo[4,3-c]pyridin-5-yl)-1-(4-chlor-phenyl)-2-oxo-ethyl]-amid; (WO 01/91752)) oder LB53280, LB53279, LB53278 oder THIQ, MB243, RY764, CHIR-785, PT-141 oder solche wie sie in WO2005060985, WO2005009950, WO2004087159, WO2004078717, WO2004078716, WO2004024720, US20050124652, WO2005051391, WO2004112793, WOUS20050222014, US20050176728, US20050164914, US20050124636, US20050130988, US20040167201, WO2004005324, WO2004037797, WO2005042516, WO2005040109, WO2005030797, US20040224901, WO200501921, WO200509184, WO2005000339, EP1460069, WO2005047253, WO2005047251, EP1538159, WO2004072076, WO2004072077, WO2006021655-57 beschrieben sind;
Orexin-Rezeptor Antagonisten (z.B. 1-(2-Methyl-benzoxazol-6-yl)-3-[1,5]naphthyridin-4-yl-harnstoff Hydrochlorid (SB-334867-A) oder solche, wie sie z. B. in WO200196302, WO200185693, WO2004085403, WO2005075458, WO2006067224 beschrieben sind); Histamin H3 Rezeptor Agonisten (z. B. 3-Cyclohexyl-1-(4,4-dimethyl-1,4,6,7-tetrahydro-imidazo[4,5-c]pyridin-5-yl)-propan-1-on Oxalsäuresalz (WO 00/63208) oder solche, wie sie in WO200064884, WO2005082893 beschrieben sind);
CRF-Antagonisten (z.B. [2-Methyl-9-(2,4,6-trimethyl-phenyl)-9H-1,3,9-triaza-fluoren-4-yl]-dipropyl-amin (WO 00/66585));
CRF BP-Antagonisten (z.B. Urocortin);
Urocortin-Agoni sten;
Agonisten des beta-3 Adrenoceptors wie z.B. 1-(4-Chlor-3-methansulfonylmethyl-phenyl)-2-[2-(2,3-dimethyl-1H-indol-6-yloxy)-ethylamino]-ethanol Hydrochlorid (WO 01/83451) oder Solabegron (GW-427353) oder N-5984 (KRP-204) oder solche, wie sie in JP2006111553 beschrieben sind;
MSH (Melanocyt-stimulierendes Hormon)-Agonisten;
MCH (melanin-konzentrierendes Hormon) Rezeptor Antagonisten (wie z. B. NBI-845, A-761, A-665798, A-798, ATC-0175, T-226296, T-71, GW-803430 oder solche Verbindungen, wie sie in WO2003/15769, WO2005085200, WO2005019240, WO2004011438, WO2004012648, WO2003015769, WO2004072025, WO2005070898, WO2005070925, WO2004039780, WO2003033476, WO2002006245, WO2002089729, WO2002002744, WO2003004027, FR2868780, WO2006010446, WO2006038680, WO2006044293, WO2006044174 beschrieben sind);
CCK-A Agonisten (wie z.B. {2-[4-(4-Chlor-2,5-dimethoxy-phenyl)-5-(2-cyclohexyl-ethyl)-thiazol-2-ylcarbamoyl]-5,7-dimethyl-indol-1-yl}-essigsäure Trifluoressigsäuresalz (WO 99/15525) oder SR-146131 (WO 0244150) oder SSR-125180) oder solchen, wie sie in WO2005116034 beschrieben sind;
Serotonin-Wiederaufnahme-Inhibitoren (z.B. Dexfenfluramine);
gemischte Sertonin- und noradrenerge Verbindungen (z.B. WO 00/71549);
5-HT-Rezeptor Agonisten z.B. 1-(3-Ethyl-benzofuran-7-yl)-piperazin Oxalsäuresalz (WO 01/09111);
5-HT2C Rezeptor Agonisten (wie z.B. Lorcaserin Hydrochlorid (APD-356) oder BVT-933 oder solche, wie sie in WO200077010, WO20077001-02, WO2005019180, WO2003064423, WO200242304, WO2005082859 beschrieben sind);
5-HT6 Rezeptor Antagonisten, wie sie z.B. in WO2005058858 beschrieben sind; Bombesin-Rezeptor Agonisten (BRS-3 Agonisten;
Galanin-Rezeptor Antagonisten;
Wachstumshormon (z.B. humanes Wachstumshormon oder AOD-9604);
Wachstumshormon freisetzende Verbindungen (6-Benzyloxy-1-(2-diisopropylamino-ethylcarbamoyl)-3,4-dihydro-1H-isochinolin-2-carbonsäuretertiärbutylester (WO 01/85695)); Growth Hormone Secretagogue Receptor Antagonisten (Ghrelin Antagonisten) wie z. B. A-778193 oder solchen, wie sie in WO2005030734 beschrieben sind;
TRH-Agonisten (siehe z.B. EP 0 462 884);
entkoppelnde Protein 2- oder 3-Modulatoren;
Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881);
Dopaminagonisten (DA-Agonisten, z.B. Bromocriptin, Doprexin);
Lipase/Amylase-Inhibitoren (z.B. WO 00/40569);
Inhibitoren der Diacylglycerol O-Acyltransferasen (DGATs) wie z. B. BAY-74-4113 oder wie z. B. in US2004/0224997, WO2004094618, WO200058491, WO2005044250, WO2005072740, JP2005206492, WO2005013907, WO2006004200, WO2006019020, WO2006064189 beschrieben;
Inhibitoren der Fettsäuresynthase (FAS) wie z.B. C75 oder solchen, wie in WO2004005277 beschrieben;
Oxyntomodulin;
Oleoyl-Estron
oder Agonisten des Schilddrüsenhormonrezeptors (thyroid hormone receptor agonists) wie z. B: KB-2115 oder solche, wie in WO20058279, WO200172692, WO200194293, WO2003084915, WO2004018421, WO2005092316 beschrieben, verabreicht.

Bei einer Ausführungsform ist der weitere Wirkstoff Varenicline Tartrate, ein partieller Agonist des alpha 4-beta 2 nikotinischen Acetylcholinrezeptors.

Bei einer Ausführungsform ist der weitere Wirkstoff Trodusquemine.

Bei einer Ausführungsform ist der weitere Wirkstoff ein Modulator des Enzyms SIRT1, eines Mitglieds der humanen Sirtuinenzymfamilie.

Bei einer Ausführungsform der Erfindung ist der weitere Wirkstoff Leptin; siehe z.B. "Perspectives in the therapeutic use of leptin", Salvador, Javier; Gomez-Ambrosi, Javier; Fruhbeck, Gema, Expert Opinion on Pharmacotherapy (2001), 2(10), 1615-1622.

Bei einer Ausführungsform ist der weitere Wirkstoff Dexamphetamin oder Amphetamin.

Bei einer Ausführungsform ist der weitere Wirkstoff Fenfluramin oder Dexfenfluramin.

Bei noch einer Ausführungsform ist der weitere Wirkstoff Sibutramin.

Bei einer Ausführungsform ist der weitere Wirkstoff Mazindol oder Phentermin.

Bei einer Ausführungsform ist der weitere Wirkstoff ein Diphenylazetidinonderivat, wie z.B. in US 6,992,067 oder US 7,205,290 beschrieben.

Bei einer Ausführungsform wird die Verbindung der Formel I in Kombination mit Ballaststoffen, vorzugsweise unlöslichen Ballaststoffen (siehe z.B. Carob/ Caromax^{®} (Zunft H J; et al., Carob pulp preparation for treatment of hypercholesterolemia, ADVANCES IN THERAPY (2001 Sep-Oct), 18(5), 230-6.) Caromax ist ein Carob enthaltendes Produkt der Fa. Nutrinova, Nutrition Specialties &Food Ingredients GmbH, Industriepark Höchst, 65926 Frankfurt / Main)) verabreicht. Die Kombination mit Caromax^{®} kann in einer Zubereitung erfolgen, oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

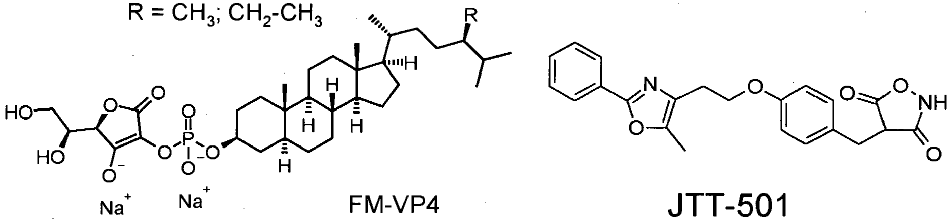

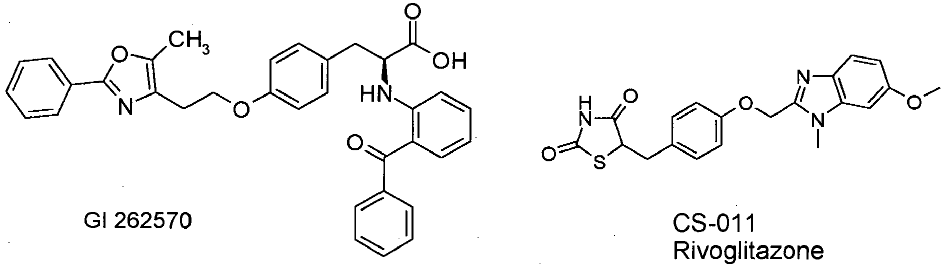

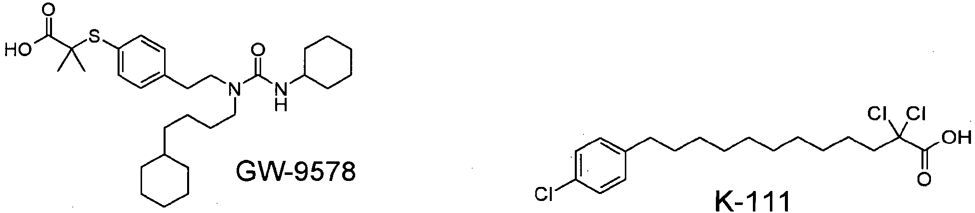

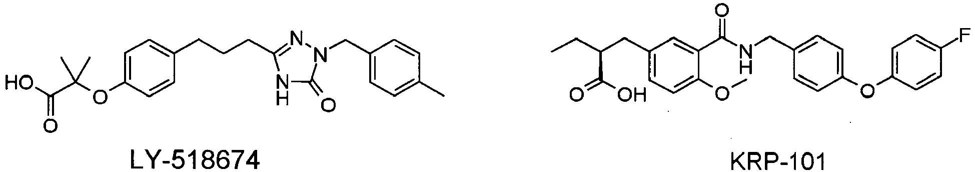

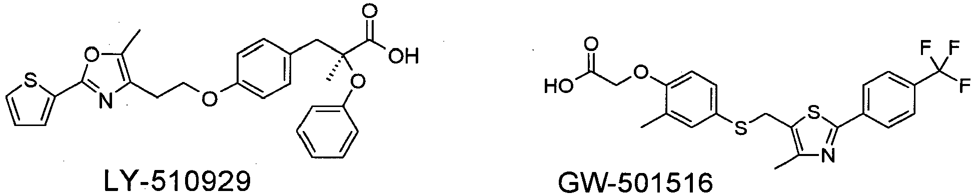

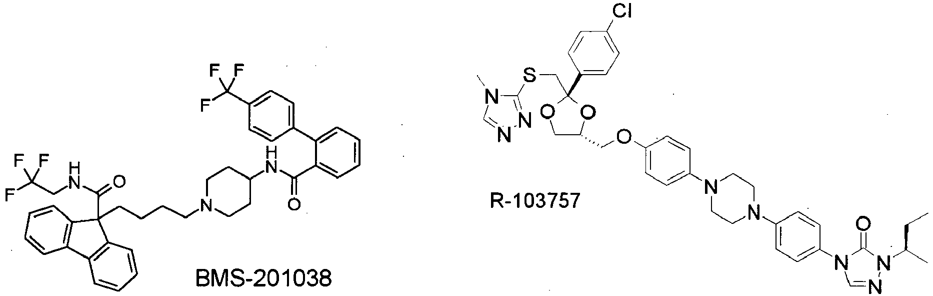

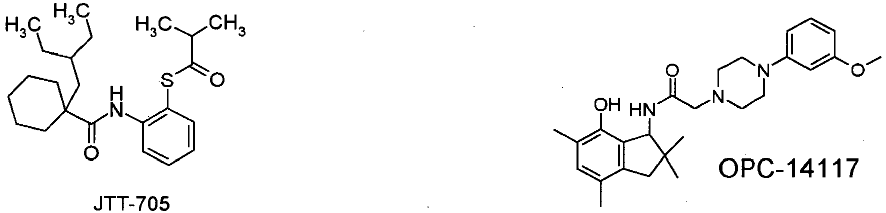

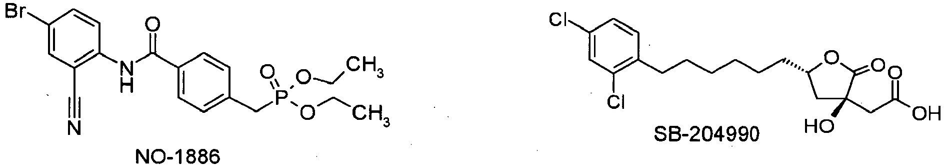

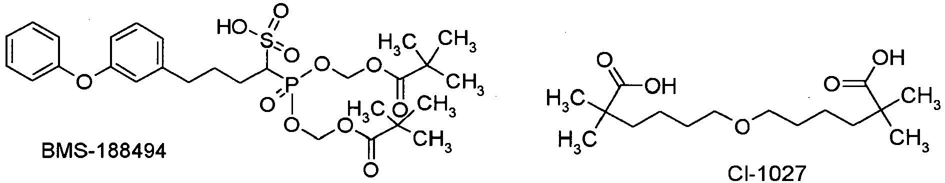

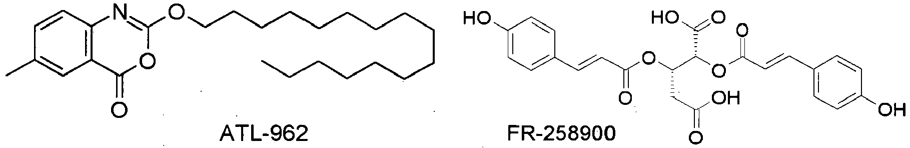

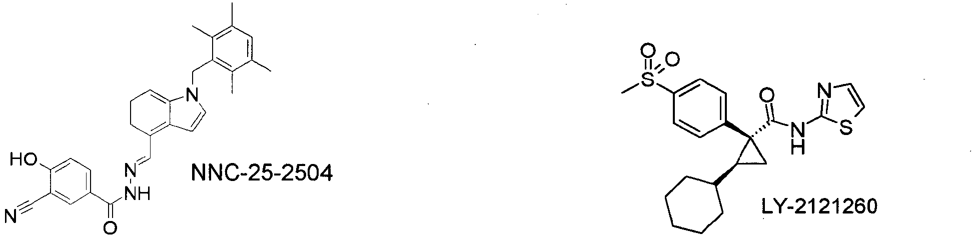

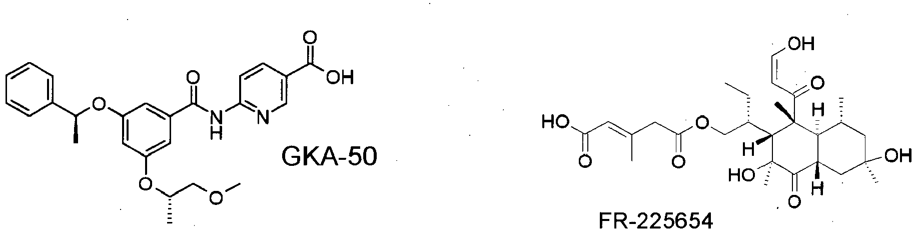

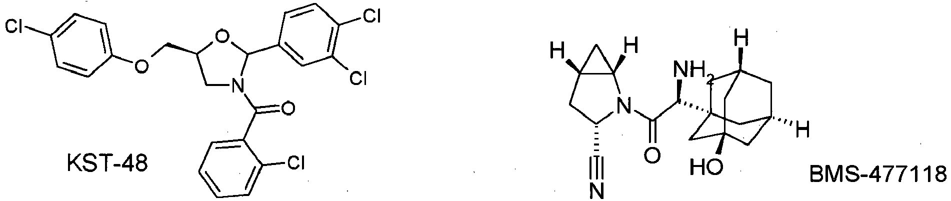

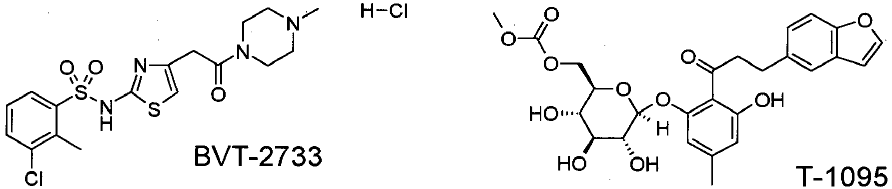

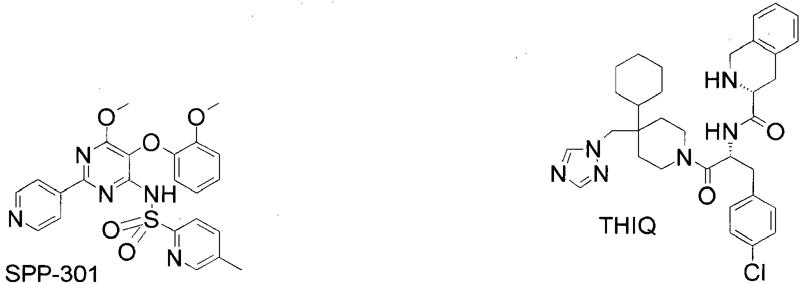

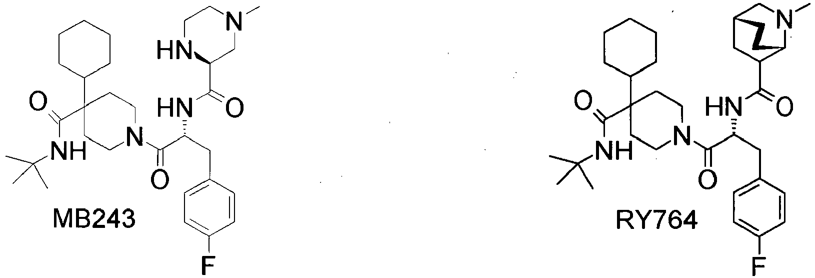

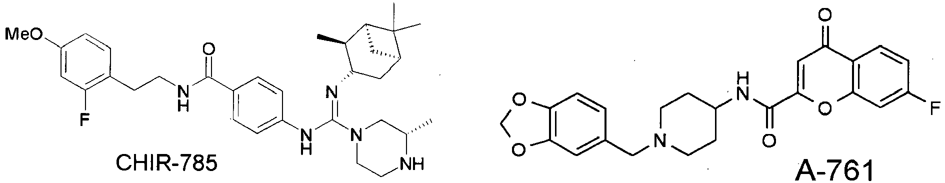

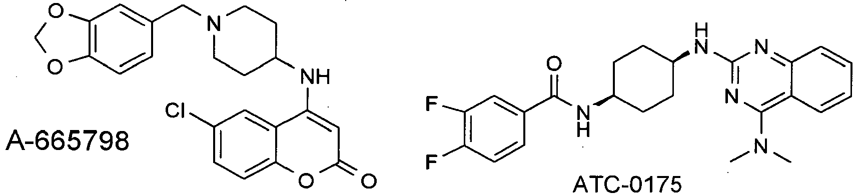

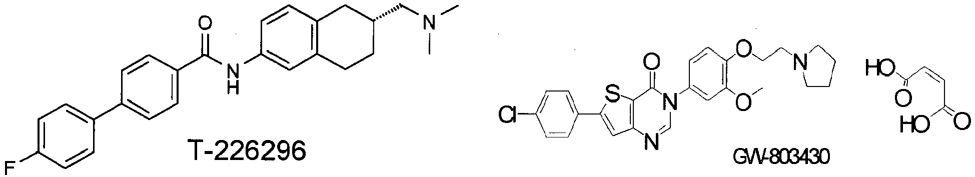

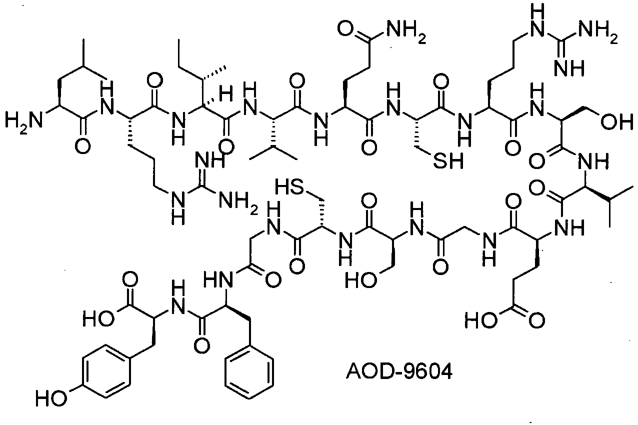

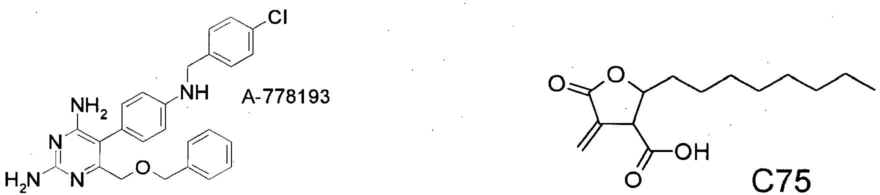

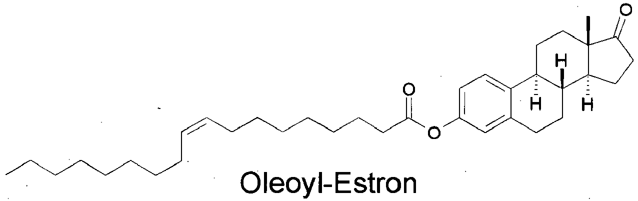

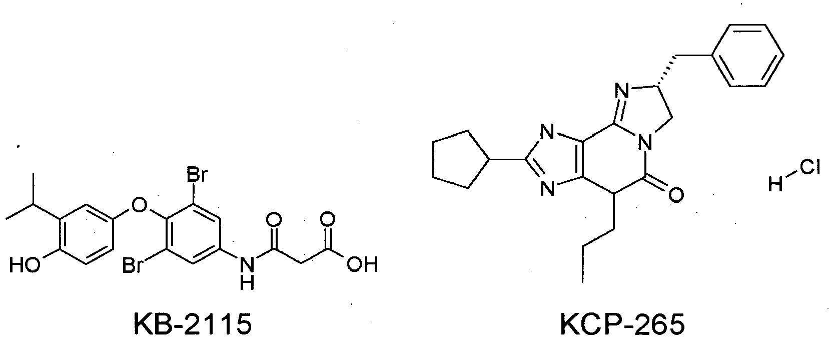

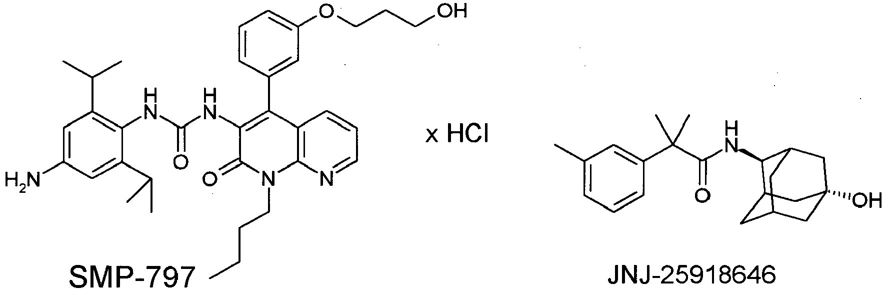

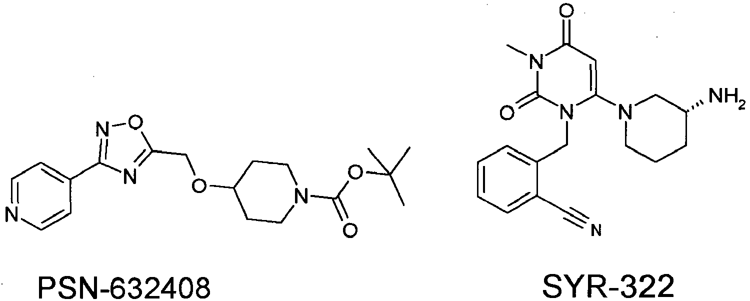

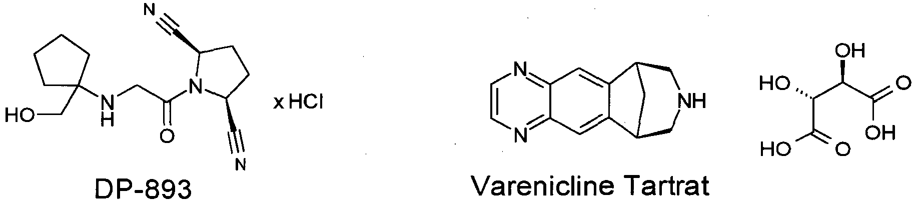

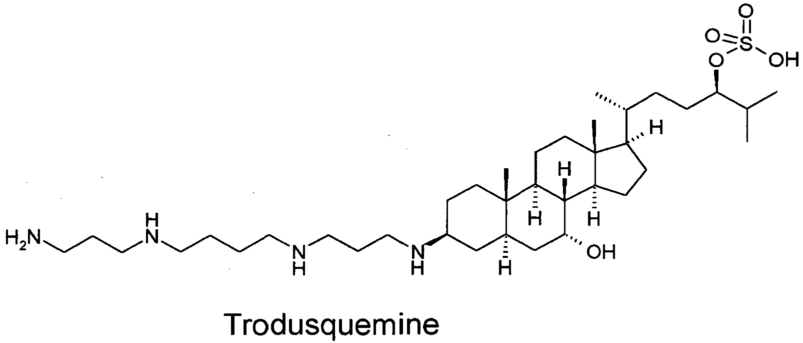

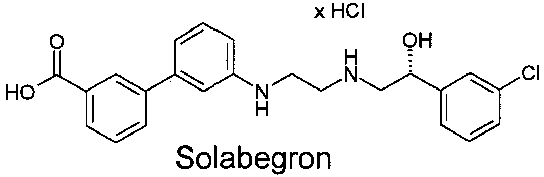

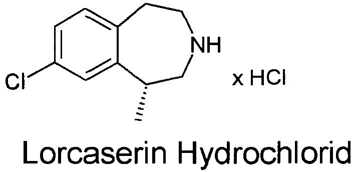

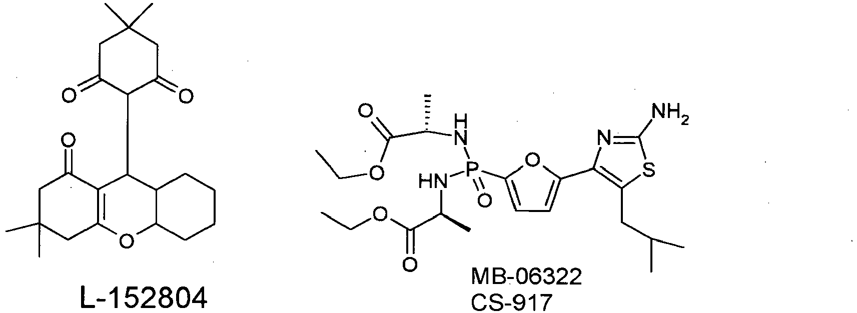

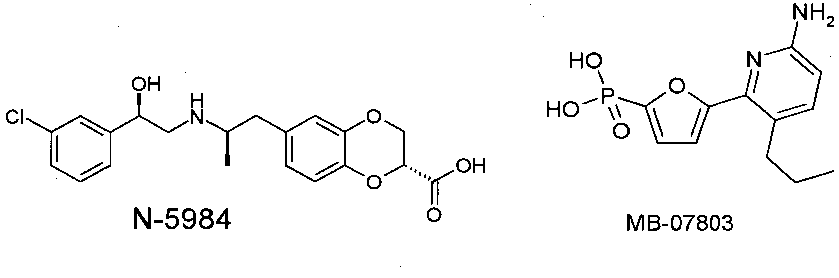

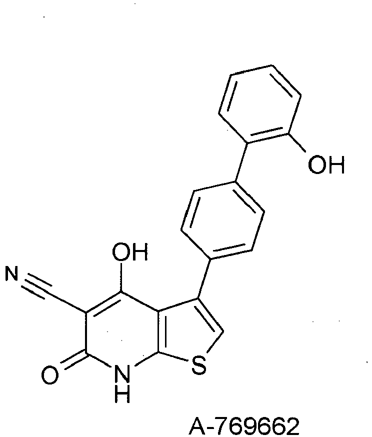

### Beispiele

Die nachfolgend aufgeführten Beispiele und Herstellungsmethoden dienen zur Erläuterung der Erfindung, ohne diese jedoch einzuschränken.

Die erfindungsgemäßen Verbindungen der Formel I können mit Hilfe von im Prinzip bekannten Reaktionen hergestellt werden. Beispielsweise wurden die Verbindungen nach folgenden allgemeinen Reaktionsschemata erhalten.

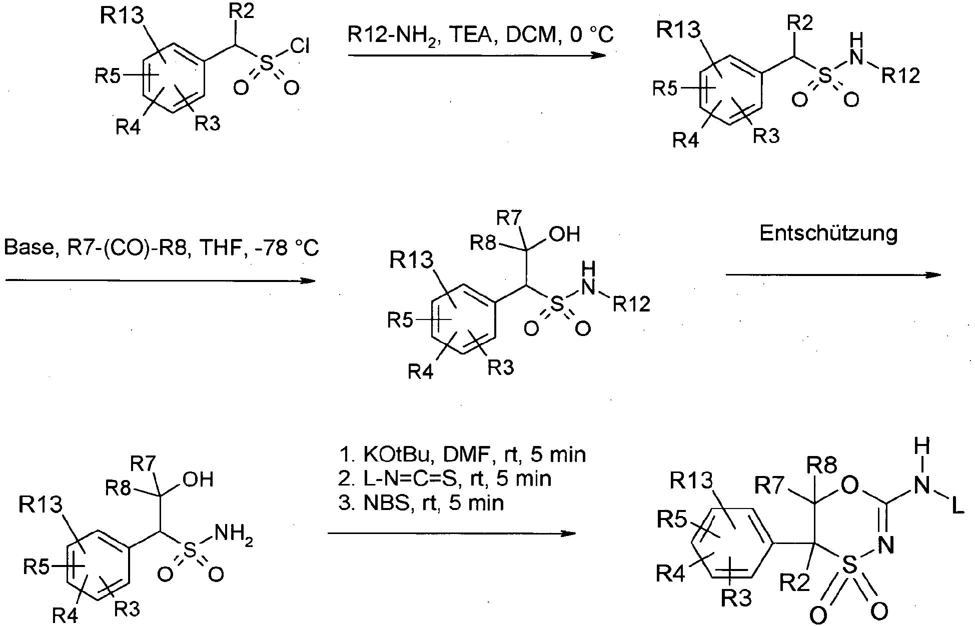

Aus einem mit einem Phenylrest und R2 substituierten Methansulfonsäurechlorid wird durch die Reaktion mit einem geeigneten Amin und Triethylamin (TEA) in einem geeigneten Lösungsmittel z.B. Dichlormethan (DCM) ein entsprechendes mit Phenyl und R2 substituiertes und mit R12 (z.B. Boc, Benzyl, 2,4-Dimethoxybenzyl) geschütztes Methansulfonsäureamid hergestellt. Durch Umsetzung mit einer geeigneten Base (z.B. Methyllithium) kann bei tiefer Temperatur dann ein Dianion erzeugt werden, das mit einem Keton (z.B. R7-(CO)-R8) oder einem Aldehyd (z.B. R7-CHO) in einem geeigneten Lösungsmittel z.B. Tetrahydrofuran (THF) zum mit R12 geschützten Hydroxysulfonamid reagiert. Durch Entschützung von R12 (z.B. bei einer Boc-Gruppe oder einer 2,4-Dimethoxybenzylgruppe durch Säurebehandlung oder bei einer Benzylgruppe durch Hydrierung) entsteht das freie Hydroxysulfonamid. Die Umsetzung des so erhaltenen Hydroxysulfonamids mit Base und einem Isothiocyanat (L-N=C=S) sowie nachfolgendem oxidativen Ringschluss mit N-Bromsuccinimid (NBS) liefert die gewünschten 4,4-dioxo-oxathiazine.

In den Fällen, in denen funktionelle Gruppen (z.B. eine Hydroxylgruppe) in geschützter Form (z.B. benzylgeschützt oder als Ester) eingebracht werden, werden diese am Schluss der Synthese durch ein geeignetes Verfahren (z.B. Hydrierung oder Reduktion) freigesetzt.

Die verwendeten Isothiocyanate werden durch die Reaktion eines primären Amins mit Thiocarbonyldiimidazol erhalten, wobei eventuell vorhandene störende funktionelle Gruppen, z.B. Hydroxylgruppen mit geeigneten Schutzgruppen, z.B. Silylether blockiert werden. Die Schutzgruppen werden am Ende der Sequenz durch geeignete Verfahren entfernt, z.B. Silylgruppen durch Behandlung mit methanlischer Salzsäure.

In den Fällen, in denen während der Synthese Diastereomere oder Racemate entstehen, können diese durch präparative HPLC voneinander getrennt werden.

Einige der eingesetzten primären Amine sind kommerziell erhältlich.

4-Fluoro-bicyclo[2.2.2]octan-1-amin kann hergestellt werden wie in der Literatur beschrieben (JOC 1982, 47, 1952-7).

Andere erfindungsgemäße Verbindungen können auf weiteren Wegen erhalten werden, die im folgenden Schema beispielhaft skizziert sind.

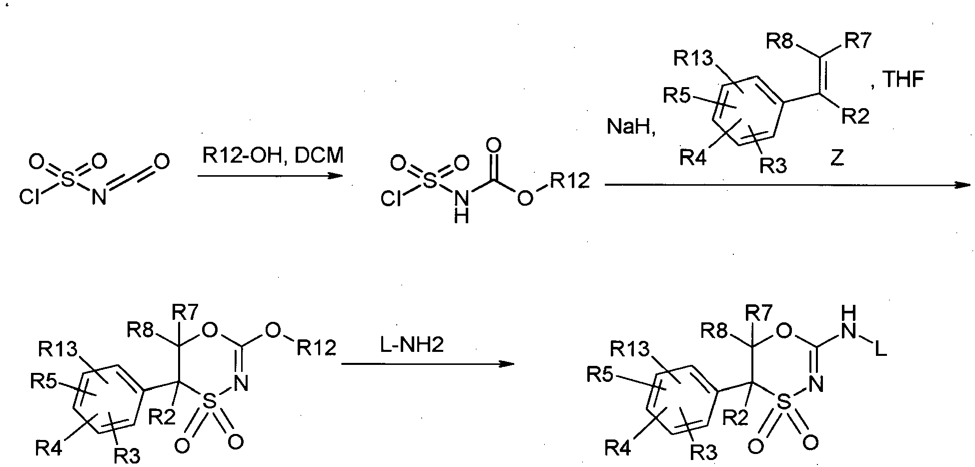

In Analogie zu einem literaturbekannten Verfahren (JACS 1972, 94, 4386-7) wird Chrosulfonylisocyanat mit einem Alkohol (R12-OH, z.B. Methanl) behandelt, wobei ein entsprechendes Carboalkoxysulfamoylchlorid entsteht (z.B. Carbomethoxysulfamoylchlorid). Dieses wird mit Natriumhydrid deprotoniert werden und die nach Chloridabspaltung entstehende Zwischenstufe (z.B. Methyl-N-Sulfonylurethan) reagiert in einer 2+4-Cycloaddition mit Alkenen der Formel Z zum alkoxysubstitierten (z.B. methoxysubstitierten) 4,4-Dioxooxathiazins umgesetzt. Die Alkoxygruppe lässt sich hier in einem geeigneten Lösemittel (z.B. Dichlormethan) durch ein Amin ersetzen, wobei die gewünschten 4,4-Dioxooxathiazine entstehen.

In den Fällen, in denen während der Synthese Diastereomere oder Racemate entstehen, können diese durch präparative HPLC voneinander getrennt werden.

Andere erfindungsgemäße Verbindungen können auf weiteren Wegen erhalten werden, die im folgenden Schema beispielhaft skizziert sind.

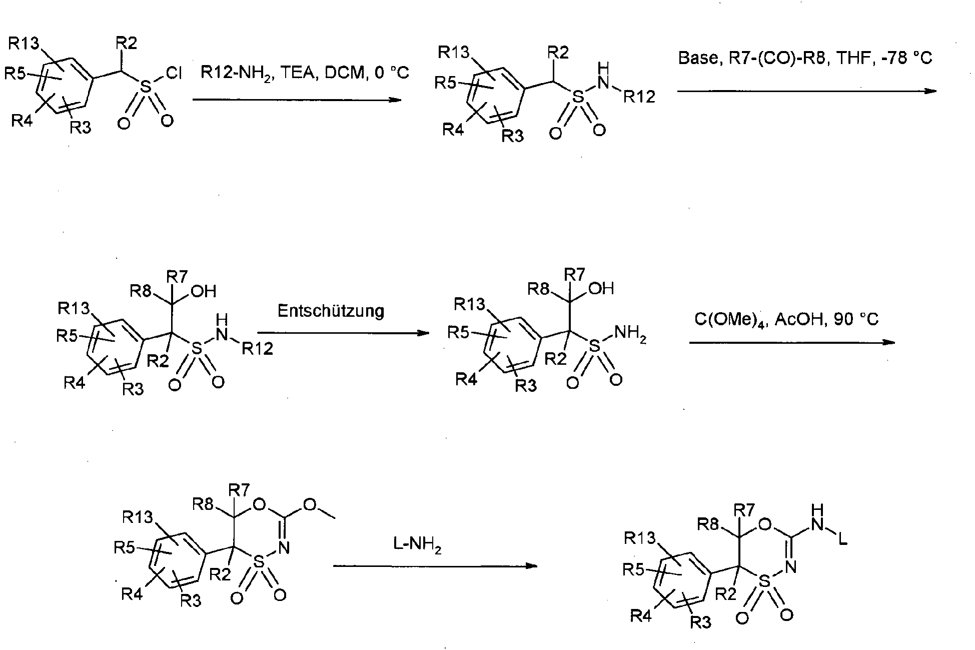

Aus einem mit Phenyl und R2 substituierten Methansulfonsäurechlorid wird durch die Reaktion mit einem geeigneten Amin und Triethylamin (TEA) in einem geeigneten Lösungsmittel z.B. Dichlormethan (DCM) ein entsprechendes mit Phenyl und R2 substituiertes und mit R12 (z.B. Boc, Benzyl, 2,4-Dimethoxybenzyl) geschütztes Methansulfonsäureamid hergestellt. Durch Behandlung mit einer geeigneten Base (z.B. Methyllithium) kann bei tiefer Temperatur dann ein Dianion erzeugt werden, das mit einem Keton (z.B. R7-(CO)-R8) oder einem Aldehyd (z.B. R7-CHO) in einem geeigneten Lösungsmittel z.B. Tetrahydrofuran (THF) zum mit R12 geschützten Hydroxysulfonamid umgesetzt wird. Durch Entschützung von R12 (z.B. bei einer Boc-Gruppe oder einer 2,4-Dimethoxybenzylgruppe durch Säurebehandlung oder bei einer Benzylgruppe durch Hydrierung) entsteht das freie Hydroxysulfonamid. Die Umsetzung des so erhaltenen Hydroxysulfonamids mit Orthokohlensäuretetramethylester liefert methoxysubstitierte 4,4-Dioxooxathiazine. Die Methoxygruppe (-O-CH₃) wird nun in einem geeigneten Lösemittel (z.B. Dichlormethan) durch ein Amin (L-NH₂) ersetzt, wobei die gewünschten 4,4-Dioxooxathiazine entstehen.

In den Fällen, in denen funktionelle Gruppen (z.B. eine Hydroxylgruppe) in geschützter Form (z.B. benzylgeschützt oder als Ester) eingebracht werden, werden diese am Schluss der Synthese durch ein geeignetes Verfahren (z.B. Hydrierung oder Reduktion) freigesetzt.

Falls das entstehende Molekül eine Halogenaryleinheit beinhaltet, kann das Halogen durch gängige metallkatalyiserte Kupplungsverfahren ersetzt werden. So kann z.B. ein Bromid mit Hilfe einer Suzuki-Reaktion oder eine Sonogashira-Reaktion oder einer palladiumkatalysierten Aminierung weiter umgesetzt werden. Weiterhin lassen sich in einigen Fällen einfache Syntheseschritte anschließen, so kann z.B. eine Dreifachbindung zur Einfachbindung hydriert werden oder ein Boc-geschütztes Amin kann entschützt und anschließend alkyliert werden.

Einige der verwendeten Amine sind kommerziell erhältlich oder können durch literaturbekannte Verfahren hergestellt werden.

Andere der verwendeten primären Amine wurden hergestellt wie im folgenden Schema beispielhaft skizziert ist.

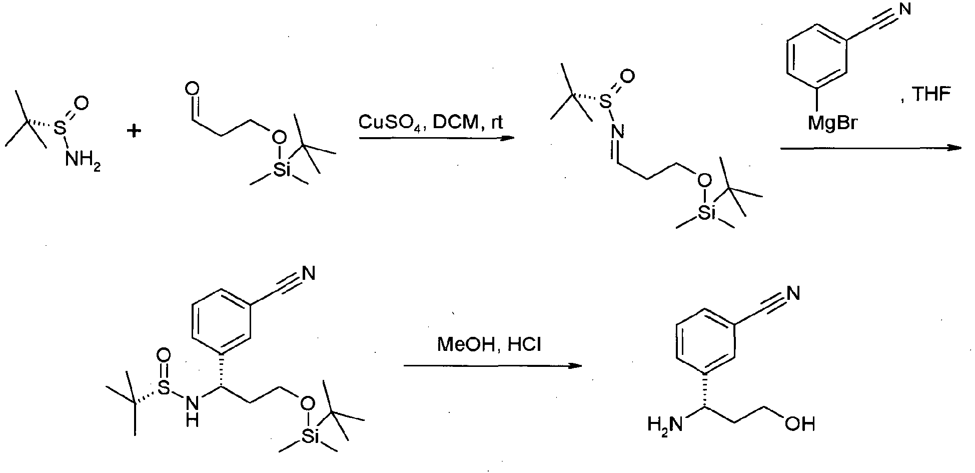

Die nachfolgend aufgeführten Beispiele dienen zur Erläuterung der Erfindung, ohne diese jedoch einzuschränken.

**Tabelle 1:**

| Beispiel | CHEMISTRY | Methode | Retentions zeit | Molgewicht (g/mol) | Name |
|---|---|---|---|---|---|
| 1 | 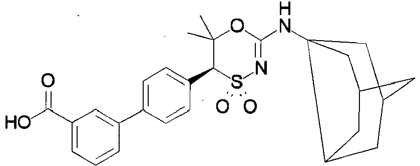 | C | 1.804 | 494.6 | 4'-[(S)-2-(Hexahydro-2,5-methano-pentalen-3a ylamino)-6,6-dimethyl-4,4-dioxo-5,6-dihydro-4H-4lambda6-[1,4,3]oxathiazin-5-yl]-biphenyl-3 carbonsäure |
| 2 | 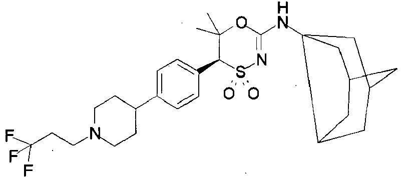 | C | 1.432 | 553.7 | ((S)-6,6-Dimethyl-4,4-dioxo-5-{4-[1-(3,3,3-trifluor-propyl)-piperidin-4-yl]-phenyl}-5,6-dihydro-4H-4lambda6-[1,4,3]oxathiazin-2-yl)-(hexahydro-2,5-methan-pentalen-3a-yl)-amin |
| 3 | 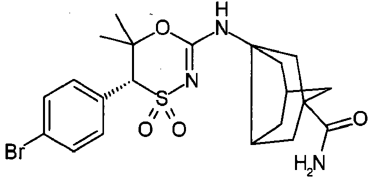 | C | 1.452 | 496.4 | 3a-[(R)-5-(4-Bromo-phenyl)-6,6-dimethyl-4,4-dioxo-5,6-dihydro-4H-4lambda6-[1,4,3]oxathiazin-2-ylamino]-hexahydro-2,5-methan-pentalen-2-carbonsäureamid * |
| 4 | 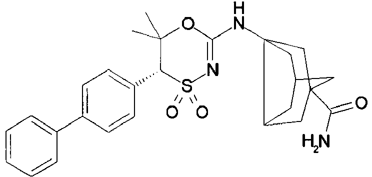 | C | 1.59 | 493.6 | 3a-((R)-5-Biphenyl-4-yl-6,6-dimethyl-4,4-dioxo-5,6-dihydro-4H-4lambda6-[1,4,3]oxathiazin-2-ylamino)-hexahydro-2,5-methan-pentalen-2-carbonsäureamid * |
| 5 | 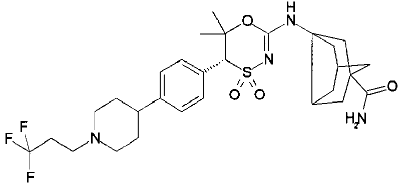 | C | 1.118 | 596.7 | 3a-((R)-6,6-Dimethyl-4,4-dioxo-5-{4-[1-(3,3,3-trifluor-propyl)-piperidin-4-yl]-phenyl}-5,6-dihydro-4H-4lambda6-[1,4,3]oxathiazin-2-ylamino)-hexahydro-2,5-methan-pentalen-2-carbonsäureamid * |
| 6 | 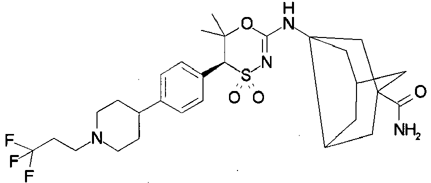 | C | 1.115 | 596.7 | 3a-((S)-6,6-Dimethyl-4,4-dioxo-5-{4-[1-(3,3,3-trifluor-propyl)-piperidin-4-yl]-phenyl}-5,6-dihydro-4H-4lambda6-[1,4,3]oxathiazin-2-ylamino)-hexahydro-2,5-methan-pentalen-2-carbonsäureamid * |

| | | | | | |
|---|---|---|---|---|---|
| * Diastereomeren Gemisch | | | | | |

### Chromatographie-Methoden:

### Methode A

LC UV/MS: Agilent 1200 Series

Säule: Mercury MS, Luna C18(2), S-3 µm, 10 x 2.0 mm

Laufmittel: 0 min 93 % H₂O (0.05 % TFA) - 1.0 min 95 % Acetonitril - 1.45 min 95 %

Acetonitril - 1.5 min 7 % Acetonitril (30 °C, Fluss 1.1 ml/min)

### Methode B

LC UV/MS: Agilent 1100 Series

Säule: Mercury MS, Luna C18(2), S-3 µm, 10 x 2,0 mm

Laufmittel: 0 min 93 % H₂O (0.05 % TFA) - 1.2 min 95 % Acetonitril - 1.4 min 95 %

Acetonitril - 1.45 min 7 % Acetonitril (30 °C, Fluss 1.1 ml / min)

### Methode C

LC UV/MS: Agilent 1100 Series

Säule: YMC J'spere ODS H80, 80 Ǻ, S-4 µm, 20 x 2.1 mm

Laufmittel: 0 min 96 % H₂O (0.05 % TFA) - 2.0 min 95 % Acetonitril - 2.4 min 95 %

Acetonitril - 2.45 min 4 % Acetonitril (30 °C, Fluss 1 ml / min)

Die Wirksamkeit der Verbindungen wurde wie folgt getestet:

### Enzymatischer 11beta-HSD1 Test:

Zum Messen der Wirkung der Verbindungen wurde ein auf SPA basierendes Nachweisverfahren (Solly *et al.* 2005) angewendet. Zunächst wurden 20 µl der humanen 11β-HSD1-Mikrosomenfraktion (0.2 µg Protein), zubereitet in 50 mM HEPES, 0.1% BSA (w/v), in eine Platte mit 384 Vertiefungen gegeben. Die Testverbindungen (0.09 µl) wurden in 100 % DMSO auf die Assayplatte übertragen. Die Reaktion wurde durch Zugabe von 20 µl [1,2-³H] Cortison (0.1 µCi / 100 mM) in Assaypuffer mit 25 mM HEPES, 100 mM KCl, 5 mM NaCl, 2 mM MgCl₂ und 0.25 mM NADPH gestartet. Die Platte wurde 1 Stunde lang bei 37 °C geschüttelt. Gleichzeitig wurde eine Stopplösung mit 20 mg / ml SPA-PVT-Perlen, 1,6 mg / ml monoklonalem Cortisol-Antikörper und 0.01 mM SSR110887 (Inhibitor aus dem Biovitrium-Patent) in 50 mM HEPES, 1 M NaCl und 1 M KCl bei Raumtemperatur gerührt. Zum Abbruch der Reaktion wurden in alle Vertiefungen jeweils 25 µl der Stopplösung gegeben. Die Platte wurde 1 weitere Stunde lang sachte bei Raumtemperatur geschüttelt und dann 1 Minute bei 500 gₐᵥ zentrifugiert, damit sich die SPA-Perlen absetzen konnten. Die Platte wurde dann in einem Wallac-1450-Microbeta-Gerät mit einem Standard-SPA-Programm gelesen (1 min Zählzeit / Vertiefung). Die Vergleichsverbindung war Glycyrrhetinsäure.

Protein und radioaktives Substrat wurden mit einem Biomek FX-Gerät (Beckman Coulter) zur Handhabung von Flüssigkeiten dispensiert. Die Testverbindungen wurden mit einem mit einem 90-nl-Pin-Tool ausgestatteten Cybi-Well (CyBio) zugesetzt.

Lit.: Solly S, Mundt SS, Zokian HJ, Juy-Fang Ding G, Hermanowski-Vosatka A, Strulovici B and Zheng W. High-throughput screening of 11β-Hydroxysteroid dehydrogenase type 1 in scintillation proximity format. Assay Drug Dev Technol 2005;3:377-384.

**Tabelle 2: Biologische Aktivität**

| Beispiel | IC₅₀ (nM) |
|---|---|
| 1 | 14 |
| 2 | 24 |
| 3 | 22 |
| 4 | 34 |
| 5 | |
| 6 | |

Aus den Messdaten ist abzulesen, dass die Verbindungen der Formel I 11beta-HSD1 (11beta-Hydroxysteroid Dehydrogenase Type 1) inhibieren und dadurch gut zur Behandlung von Hyperglykämie, Insulin Resistenz, Diabetes, Adipositas, Lipidstoffwechselstörungen und anderen Erkrankungen geeignet sind.

Nachfolgend wird die Herstellung einiger Beispiele detailliert beschrieben, die übrigen Verbindungen der Formel I wurden analog erhalten:

### Experimenteller Teil:

C-(4-Bromo-phenyl)-N-(2,4-dimethoxy-benzyl)-methansulfonamid

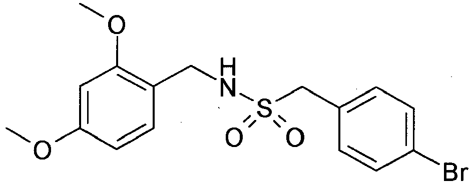

Unter Eiskühlung wurden 20.0 g 4-bromobezylsulfonyl chloride in 200 ml Dichlormethan vorgelegt und langsam eine Lösung aus 24 ml 2,4-Dimethoxybenzylamin in 100 ml Dichlormethan zugetropft. Zur verbesserten Rührbarkeit wurden weitere 100 ml Dichlormethan zugegeben und die Reaktionsmischung auf Raumtemperatur kommen gelassen. Nach 2 Stunden Rühren wurde die Reaktionsmischung mit 100 ml Wasser, mit 100 ml 0.2 N wässriger Salzsäure, mit 100 ml gesättigter wässriger Natriumhydrogencarbonat-Lösung und abschließend noch einmal mit 100 ml Wasser gewaschen. Die organische Phase wurde über Na₂SO₄ getrocknet, einrotiert und im Hochvakuum getrocknet. Der Rückstand (30 g) wurde ohne weitere Reinigung in die nächste Reaktion eingesetzt.

### 1-(4-Bromo-phenyl)-2-hydroxy-2-methyl-propan-1-sulfonsäure 2,4-dimethoxy-benzylamid

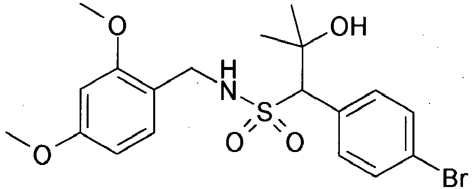

Unter Inertgas wurden 28 g C-(4-Bromo-phenyl)-N-(2,4-dimethoxy-benzyl)-methansulfonamid in 250 ml THF vorgelegt und anschließend bei einer Temperatur von -78 °C 93 ml einer 1.6 N Methyllithium-Lösung in Hexan tropfenweise zugegeben und 5 Minuten unter Eiskühlung gerührt. Anschließend wurde die Reaktionslösung erneut auf -78 °C abgekühlt und 20 ml Aceton zugegeben. Die Mischung wurde auf Raumtemperatur kommen gelassen und 30 Minuten gerührt. Die Reaktionslösung wurde mit 13 ml Trifluoressigsäure und 500 ml Ethylacetat versetzt, mit 300 ml einer gesättigten wässrigen Natriumhydrogencarbonat-Lösung und 300 ml einer gesättigten wässrigen Natriumchlorid-Lösung gewaschen, die organische Phase über Na₂SO₄ getrocknet und einrotiert. Der Rückstand wurde ohne weitere Reinigung in die nächste Reaktion eingesetzt.

### 1-(4-Bromo-phenyl)-2-hydroxy-2-methyl-propan-1-sulfonsäureamid

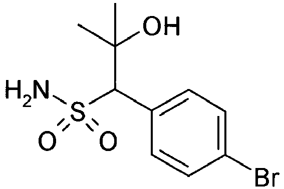

Eine Lösung aus 1-(4-Bromo-phenyl)-2-hydroxy-2-methyl-propan-1-sulfonsäure 2,4-dimethoxy-benzylamid aus obiger Reaktion in 150 ml Dichlormethan wurde mit 50 ml Trifluoressigsäure versetzt und 17 Stunden bei Raumtemperatur gerührt. Die Ansatzlösung wurde 2 x mit 100 ml Toluol koevaporiert und das Rohprodukt mittels Normalphasenchromatographie über den Flashmaster mit einem n-Heptan/Ethylacetat-Gradienten gereinigt. Die produkthaltigen Fraktionen wurden vereinigt und einrotiert. Man erhielt so das Produkt (16 g) mit dem Molekulargewicht 308.2 g / mol (C₁₀H₁₄BrNO₃S).

### 5-(4-Brom-phenyl)-2-methoxy-6,6-dimethyl-5,6-dihydro-[1,4,3]oxathiazin 4,4-dioxid

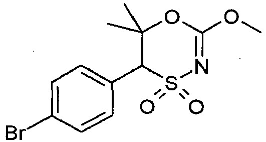

Eine Mischung aus 500 mg 1-(4-Brom-phenyl)-2-hydroxy-2-methyl-propan-1-sulfonsäureamid in 2 ml Tetramethoxymethan und 0.5 ml Essigsäure wurde für 3 Stunden bei 100 °C gerührt. Nach dem Entfernen der Lösungsmittel unter vermindertem Druck wurde der Rückstand (565 mg) ohne weitere Reinigung in die nächste Reaktion eingesetzt.

Die Verbindung kann durch chirale Chromatographie in die Enantiomere (R)-5-(4-Bromo-phenyl)-2-methoxy-6,6-dimethyl-5,6-dihydro-[1,4,3]oxathiazin 4,4-dioxid und (S-5-(4-Bromo-phenyl)-2-methoxy-6,6-dimethyl-5,6-dihydro-[1.,4,3]oxathiazin 4,4-dioxid aufgetrennt werden.

4'-[(S)-2-(Hexahydro-2,5-methan-pentalen-3a-ylamino)-6,6-dimethyl-4,4-dioxo-5,6-dihydro-4H-4lambda*6*-[1,4,3]oxathiazin-5-yl]-biphenyl-3-carbonsäure

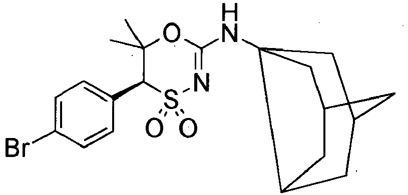

Eine Lösung aus 411 mg (S)-5-(4Brom-phenyl)-2-methoxy-6,6-dimethyl-5,6-dihydro-1,4,3-oxathiazin-4,4-dioxid und 270 mg 3-Noradamantanamin (170 mg) in 5 ml Methylenchlorid wurde einrotiert und der Rückstand über Nacht bei Raumtemperatur stehen gelassen. Das Rohprodukt wurde mittels Normalphasenchromatographie über den Flashmaster mit einem n-Heptan/Ethylacetat-Gradienten gereinigt. Die produkthaltigen Fraktionen wurden vereinigt und einrotiert. Man erhielt so das Produkt (431 mg) mit dem Molekulargewicht 453.4 g / mol (C₂₀H₂₅BrN₂O₃S).

### 4'-[(S)-2-(Hexahydro-2,5-methan-pentalen-3a-ylamino)-6,6-dimethyl-4,4-dioxo-5,6-dihydro-4H-4lambda*6*-[1,4,3]oxathiazin-5-yl]-biphenyl-3-carbonsäure

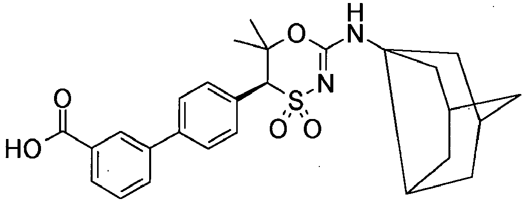

Unter Inertgas wurden zu einer Mischung aus 79 mg 3-Carboxyphenylboronsäure, 150 mg 4'-[(S)-2-(Hexahydro-2,5-methan-pentalen-3a-ylamino)-6,6-dimethyl-4,4-dioxo-5,6-dihydro-4H-4lambda*6*-[1,4,3]oxathiazin-5-yl]-biphenyl-3-carbonsäure und 380 mg Cäsiumcarbonat in 3 ml Dioxan und 1 ml Wasser 40 mg CTC-Q-PHOS gegeben. Nach 40 Minuten Rühren bei 62 °C wurde die Reaktion auf Raumtemperatur abkühlen gelassen, mit 50 ml Ethylacetat versetzt und die organische Phase mit 20 ml Wasser und 20 ml gesättigter wässriger Natriumchlorid-Lösung gewaschen. Die organische Phase wurde über MgSO₄ getrocknet und einrotiert. Das Rohprodukt wurde mittels Normalphasenchromatographie über den Flashmaster mit einem n-Heptan/Ethylacetat-Gradienten gereinigt. Die produkthaltigen Fraktionen wurden vereinigt und einrotiert.. Das Rohprodukt wurde im Reinigungslabor über präparative HPLC gereinigt. Die produkthaltigen Fraktionen wurden vereinigt, das Acetonitril unter vermindertem Druck entfernt, der wässrige Rückstand mit gesättigter wässriger Natriumhydrogencarbonat-Lösung basisch gestellt und mit Dichlormethan extrahiert. Die organische Phase wurde über MgSO₄ getrocknet und einrotiert, der Rückstand erneut in einer Mischung aus Acetonitril und Wasser aufgenommen und lyophilisiert. Man erhielt so das Produkt (49 mg) mit dem Molekulargewicht 494.6 g / mol (C₂₇H₃₀N₂O₅S); MS (ESI): m / e = 495 (M+H⁺).

Analog wurden folgende Produkte erhalten:
3a-[(R)-5-(4-Bromo-phenyl)-6,6-dimethyl-4,4-dioxo-5,6-dihydro-4H-4lambda*6*-[1,4,3]oxathiazin-2-ylamino]-hexahydro-2,5-methan-pentalen-2-carbonsäureamid 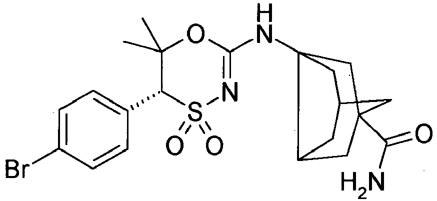
3a-((R)-5-Biphenyl-4-yl-6,6-dimethyl-4,4-dioxo-5,6-dihydro-4H-4lambda*6*-[1,4,3]oxathiazin-2-ylamino)-hexahydro-2,5-methan-pentalen-2-carbonsäureamid 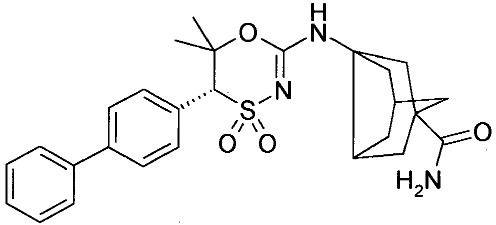
4-{4-[(R)-2-(2-Carbamoyl-hexahydro-2,5-methan-pentalen-3a-ylamino)-6,6-dimethyl-4,4-dioxo-5,6-dihydro-4H-4lambda*6*-[1,4,3]oxathiazin-5-yl]-phenyl}-3,6-dihydro-2H-pyridine-1-carbonsäurebutyl ester

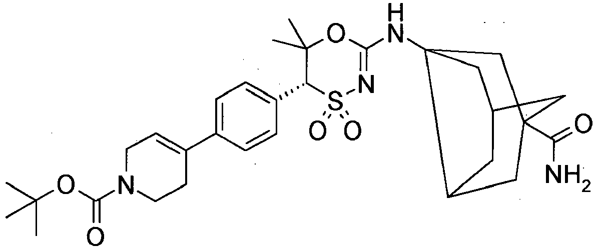

Eine Lösung aus N-boc-4-(4,4,5,5-Tetramethyl-[1,3,2]dioxaborolan-2-yl)-3,6-dihydro-2H-pyridin (112 mg), Cäsiumcarbonat (212 mg) und 3a-[(R)-5-(4-Bromo-phenyl)-6,6-dimethyl-4,4-dioxo-5,6-dihydro-4H-4lambda*6*-[1,4,3]oxathiazin-2-ylamino]-hexahydro-2,5-methan-pentalen-2-carbonsäureamid (150 mg) in Dioxan (2 ml) / Wasser (1 ml) wurde mit Argon zwanzig Minuten gespült. Dann wurden CTC-Q-PHOS (43 mg) und Bis(dibenzylideneaceton)palladium(17 mg) zugegeben und dreißig Minuten bei 65 °C gerührt. Anschließend wurde mit Ethylacetat (20 ml) versetzt und mit Wasser (10 ml) gewaschen. Die organische Phase wurde über eine Kieselgurkartusche getrocknet, filtriert und im Vakuum konzentriert. Anschließend wurde säulenchromatographisch (Ethylacetat / Heptan) gereinigt. Man erhielt so das Produkt (140 mg) mit dem Molekulargewicht 598.8 g / mol (C₃₁H₄₂N₄O₆S).

### 3a-[(R)-6,6-Dimethyl-4,4-dioxo-5-(4-piperidin-4-yl-phenyl)-5,6-dihydro-4H-4lambda*6*-[1,4,3]oxathiazin-2-ylamino]-hexahydro-2,5-methan-pentalen-2-carbonsäureamid

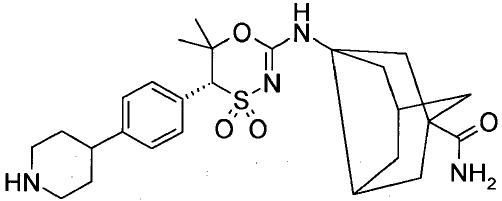

Eine Lösung von 4-{4-[(R)-2-(2-Carbamoyl-hexahydro-2,5-methan-pentalen-3a-ylamino)-6,6-dimethyl-4,4-dioxo-5,6-dihydro-4H-4lambda*6*-[1,4,3]oxathiazin-5-yl]-phenyl}-3,6-dihydro-2H-pyridine-1-carbonsäurebutyl ester (100 mg) in Methanl (10 mL) wurde mit Palladium auf Kohle (10%, 100 mg) versetzt und 1,5 h in einer Wasserstoffatmosphäre gerührt. Die Mischung wurde filtriert und eingeengt. Der Rückstand wurde in 5 mL Dichlormethan gelöst und mit 1 mL Trifluoressigsäure versetzt. Nach 1 h Rühren bei Raumtemperatur wurde das Lösungsmittel im Vakuum entfernt und entstandener TFA Ester durch Behandeln mit 1 N HCl gespalten. Extraktion mit Ethylacetat lieferte das Rohprodukt, das ohne weitere Reinigung eingesetzt wurde.

### 3a-((R)-6,6-Dimethyl-4,4-dioxo-5-{4-[1-(3,3,3-trifluoro-propyl)-piperidin-4-yl]-phenyl}-5,6-dihydro-4H-4lambda*6*-[1,4,3]oxathiazin-2-ylamino)-hexahydro-2,5-methan-pentalen-2-carboxylic acid amide

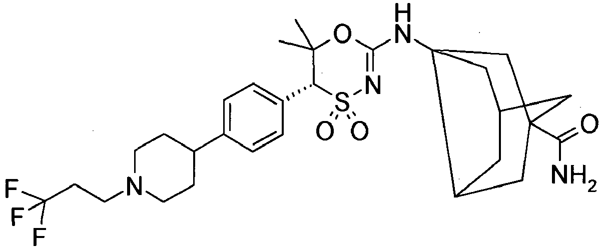

Zu einer Lösung aus 3a-[(R)-6,6-Dimethyl-4,4-dioxo-5-(4-piperidin-4-yl-phenyl)-5,6-dihydro-4H-4lambda*6*-[1,4,3]oxathiazin-2-ylamino]-hexahydro-2,5-methan-pentalen-2-carbonsäureamid (73 mg)in DMF (1,5 mL) wurde DIPEA (75 mg) und 3-Brom-1,1,1-trifluorpropan (500 mg) gegeben und die Mischung wurde über Nacht gerührt. Die entstandene Mischung wurde im Reinigungslabor über präparative HPLC gereinigt. Man erhielt so das Produkt mit dem Molekulargewicht 596.7 g / mol (C₂₉H₃₉F₃N₄O₄S); MS (ESI): m / e = 597 (M+H⁺).

Analog wurden folgende Produkte erhalten:
3a-((S)-6,6-Dimethyl-4,4-dioxo-5-{4-[1-(3,3,3-trifluoro-propyl)-piperidin-4-yl]-phenyl}-5,6-dihydro-4H-4lambda*6*-[1,4,3]oxathiazin-2-ylamino)-hexahydro-2,5-methan-pentalen-2-carboxylic acid amide 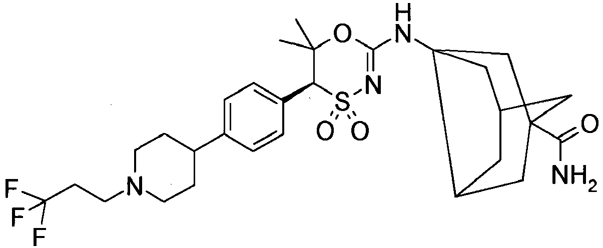
((S)-6,6-Dimethyl-4,4-dioxo-5-{4-[1-(3,3,3-trifluoro-propyl)-piperidin-4-yl]-phenyl}-5,6-dihydro-4H-4lambda*6*-[1,4,3]oxathiazin-2-yl)-(hexahydro-2,5-methan-pentalen-3a-yl)-amine 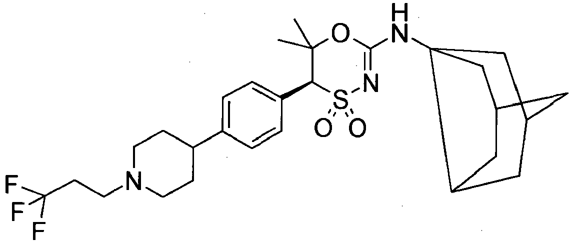
5-Phenyl-adamantan-2-on

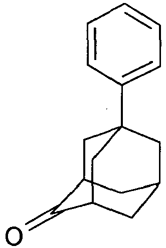

WO2007/113634A1 zeigt die chemische Umsetzung, allerdings wurde hier die Aufarbeitung (Kristallisation) optimiert. Die Umsetzung liefert das Produkt so in größerer Reinheit bei vergleichbarer Ausbeute.

(3R,7R)-5-Hydroxy-adamantan-2-on (25 g) wurde in Benzol (200 ml) gelöst. Trifluormethansulfonsäure (13.1 ml) wurde so zugegeben, dass die Reaktion bei Raumtemperatur rührte. Anschließend wurde die Reaktion am Rückfluss gerührt. Nach erneuter Zugabe von Trifluormethansulfonsäure (2 ml) wurde die Reaktion erneute am Rückfluss gerührt. Nach Zugabe von tert-Butylmethylether wurde mehrmals mit Wasser gewaschen. Die resultierende Lösung wurde nach Filtration vollständig eingeengt. Der Rückstand wurde in n-Heptan aufgenommen, heiß filtriert und dann kristallisiert. Man erhielt 5-Phenyl-adamantan-2-on (26.9 g) als weißen Feststoff.

### 2-Methyl-5-phenyl-adamantan-2-ol

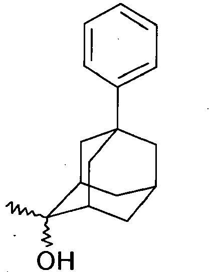

Eine Herstellung ist in WO2007/113634A1 beschrieben. Die hier beschriebene Methode ist sicherer, da der direkte Umgang mit Methylmagnesiumbromid / chlorid vermieden wird. Das Grignard-Reagenz wird in-situ gebildet. Dies erleichtert größere Ansätze (im Labormaßstab) erheblich.

5-Phenyl-adamantan-2-on (301 g) wurde in einem Gemisch von THF (4.25 1) und tert-Butylmethylether (850 ml) gelöst. Nach Zugabe von Iodmethan (566 g) wurde Magnesium (97 g) zugegeben. Die Reaktion rührte anschließend bis zum vollständigen Umsatz, wobei eine Te2.5 Imperatur von 55°C nicht überschritten wurde. Nach vollständigem Umsatz wurde mit tert-Butylmethylether (31) und Wasser (300 ml) verdünnt. Durch Zugabe von Salzsäure (32%ig) wurde ein pH-Wert von pH2 eingestellt. Nach erneuter Wasserzugabe und anschließender Phasentrennung wurde die org. Phase mehrmals mit Wasser extrahiert. Die org. Lösung wurde mit Aktivkohle geklärt und anschließend im Vakuum vollständig eingeengt. Man erhielt 2-Methyl-5-phenyl-adamantan-2-ol (312.2 g) als Öl.

### 1-(2-Phenyl-hexahydro-2,5-methan-pentalen-3a-yl)-ethanon

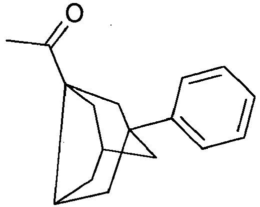

Die Umsetzung ist in WO2007/113634A1 mit schlechterer Ausbeute (59% vs 98%) beschrieben.

Eine Lösung von 2-Methyl-5-phenyl-adamantan-2-ol (300 g) in THF (710 ml) und Essigsäure (160 ml) wurde bei 0°C zu einer Lösung (8.3%, 3330 ml) von Natriumhypochlorid in Wasser gegeben. Nach Zugabe von n-Tetrabutylammoniumiodid (25.6 g) wurde innerhalb von 90 Minuten auf 25°C erwärmt. Nach vollständigem Umsatz und Phasentrennung wurde die org. Phase mehrmals mit Wasser gewaschen. Nach vollständigem Einengen der org. Phase wurde der Rückstand in Methanol (525 ml) aufgenommen. Es wurde Kaliumhydroxid (82 g) zugegeben und das Gemisch bei Raumtemperatur gerührt. Nach Zugabe von tert-Butylmethylether wurde mehrmals mit Wasser gewaschen. Die org. Lösung wurde mit Aktivkohle geklärt und im Vakuum vollständig eingeengt. Man erhielt 1-(2-Phenyl-hexahydro-2,5-methan-pentalen-3a-yl)-ethanon (320 g) als Öl.

### 2-Phenyl-hexahydro-2,5-methan-pentalen-3a-carbonsäure

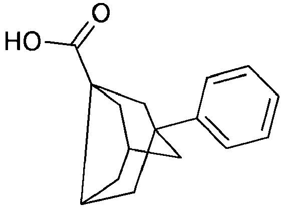

Die Umsetzung ist in WO2007/113634A1 mit schlechterer Ausbeute (64% vs 89%) beschrieben.

Zu einer auf -22°C gekühlten Lösung von Natriumhydroxid (724 g) in Wasser (4.81) wurde Dioxan (1.8 l) gegeben. Brom (578 g) wurde so in diese Lösung dosiert, dass eine Temperatur von -15°C nicht überschritten wurde. Zu dieser Lösung wurde eine Lösung von 1-(2-Phenyl-hexahydro-2,5-methano-pentalen-3a-yl)-ethanone (290 g) in Dioxan (600 ml) dosiert und man ließ die Reaktion langsam auf Raumtemperatur erwärmen. tert-Butylmethylether wurde zugeben. Anschließend wurde Wasser (2.5 l) zudosiert. Nach Phasentrennung wurde die wäßr. Phase mit tert-Butylmethylether gewaschen. Anschließend wurde nach Zugabe von tert-Butylmethylether (5 l) mit Salzsäure (32%) ein pH-Wert von pH 1.5 eingestellt. Nach Phasentrennung wurde die org. Phase mehrmals mit Wasser gewaschen und mit Aktivkohle geklärt. Durch Destillation im Vakuum unter Zugabe von Diisopropylether wurde das Lösungsmittel gewechselt. Nach Ausrühren mit Diisopropylether am Rückfluss wurde 2-Phenyl-hexahydro-2,5-methan-pentalen-3a-carbonsäure (292 g) als Feststoff mit 95%iger Reinheit isoliert.

### (2-Phenyl-hexahydro-2,5-methan-pentalen-3a-yl)-carbaminsäure tert-butyl ester

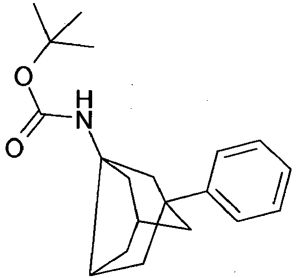

2-Phenyl-hexahydro-2,5-methan-pentalen-3a-carboxylsäure (50 g) wurde in t-Butanol (1 1) gelöst und zu dieser Lösung Triethylamin (51 g) gegeben. Diphenylphosphorylazid (56 g) wurde bei Raumtemperatur portionsweise zugegeben. Die Reaktion wurde zunächst bei Raumtemperatur und anschließend am Rückfluss gerührt. Nach Entfernen des t-Butanol wurde der Rückstand in Wasser (800 ml) aufgenommen. Der ausgefallene Feststoff wurde abfiltriert und mehrmals mit Wasser gewaschen. Nach Trocknung im Vakuum wurde der Feststoff in tert-Butylmethylether aufgenommen. Der sich dabei bildende Feststoff wurde abfiltriert. Das Filtrat wurde im Vakuum vollständig eingeengt und anschließend mit Diisopropylether verrührt. Der Feststoff wurde abgesaugt und mit einem Gemisch von Diisopropylether und n-Heptan gewaschen. Nach Trocknung erhielt man (2-Phenyl-hexahydro-2,5-methan-pentalen-3a-yl)-carbaminsäure tert-butyl ester (83.9 g).

### 3a-tert-Butoxycarbonylamino-hexahydro-2,5-methan-pentalen-2-carbonsäure

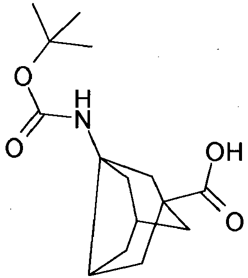

(2-Phenyl-hexahydro-2,5-methan-pentalen-3a-yl)-carbaminsäure tert-butyl ester (12 g) wurde in Acetonitril (240 ml), Tetrachlorkohlenstoff (240 ml) und Wasser (360 ml) vorgelegt, dann Rutheniumchlorid (0.4 g) zugegeben. Anschließend wurde Natriumperiodat (120 g) portionsweise zudosiert. Die Reaktion wurde bei Raumtemperatur nachgerührt und dann mit tert-Butylmethylether verdünnt. Nach Phasentrennung wurde die org. Phase mehrmals mit Wasser gewaschen und danach mit Natronlauge (0.1 M, 100 ml) extrahiert. Anschließend wurde die wäßr. Phase mit tert-Butylmethylether gewaschen und dann in Gegenwart von tert-Butylmethylether mit Salzsäure (0.5 M) auf einen pH-Wert von pH 3-4 eingestellt. Nach Phasentrennung wurde die org. Phase mehrmals mit Wasser gewaschen und dann im Vakuum vollständig eingeengt. Der Rückstand wurde mit einem Gemisch von Acetonitril und Diisopropylether verrührt. Nach Absaugen und Trocknung erhielt man 3a-tert-Butoxycarbonylamino-hexahydro-2,5-methano-pentalene-2-carboxylsäure (6.6 g) als weißen Feststoff.

### (2-Carbamoyl-hexahydro-2,5-methan-pentalen-3a-yl)-carbaminsäure tert-butyl ester

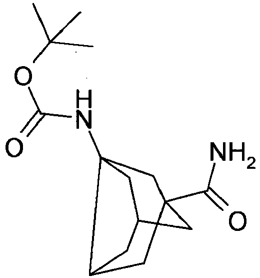

3a-tert-Butoxycarbonylamino-hexahydro-2,5-methan-pentalen-2-carbonsäure wurden in DMF (200 ml) gelöst, DIPEA (10,6 g) und Ammoniak (6,1 ml, 7M in MeOH) zugegeben. Anschließend wurde HATU (14, 8 g) zugestzt und bei Raumtemperatur 2h gerührt. Die Reaktionslösung wurde bis auf 50ml Volumen einrotiert, in 400ml verdünnte NaHCO₃-Lsg. (300ml H₂O + 100 ml gesättigter NaHCO₃-Lsg.) gegossen und 3 mal mit 100ml EtOAc extrahiert. Dann über MgSO₄ getrocknet, filtriert und einrotiert. Rückstand in DMF gelöst und über Umkehrphase (Biotage) gereinigt. Die Wertfraktionen wurden vereinigt, Acetonitril abgedampft und den wässrigen Rückstand gefriergetrocknet. Der Rückstand (1,81 g) wurde ohne weiter Aufarbeitung weiterverwendet.

### 3a-Amino-hexahydro-2,5-methan-pentalen-2-carbonsäureamid

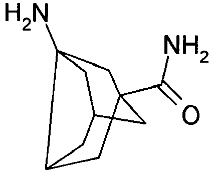

(2-Carbamoyl-hexahydro-2,5-methan-pentalen-3a-yl)-carbaminsäure tert-butyl ester (1,8 g) in Dichlormethan (50 ml) gelöst, mit 11 equivalente HCl (4M in Dioxan) versetzt und bei Raumtemperatur über Nacht gerührt. LCMS: Amin ist vollständig entschützt. Die Reaktionslösung wurde einrotiert, das HCl-Salz des Amins mit 50ml ges. Na₂CO₃-Lsg + 3ml 2M NaOH freigesetzt und insgesamt 8 mal mit 20ml Dichlormethan und 1 mal mit EtOAc extrahiert. Die organischen Lösungen wurden vereinigt, über MgSO₄ getrocknet, filtriert, einrotiert und im Hochvakuum getrocknet. Der Rückstand (0,91 g) wurde ohne weitere Aufarbeitung weiterverwendet.

## Patentansprüche

1. Verbindungen der Formel I, 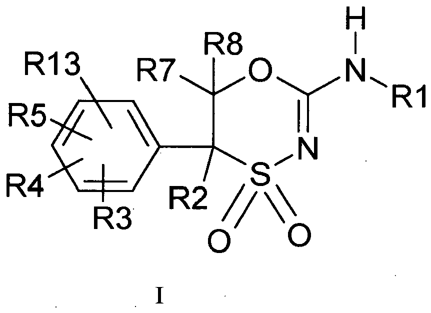 worin bedeuten
R1 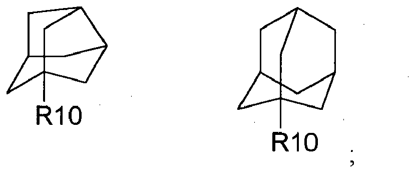
R2 H, F, (C₁-C₃)-Alkyl wobei der Alkylrest ein bis 3-fach mit Fluor substituiert sein kann;
R3, R4, R5, R13 unabhängig voneinander
H, F, Cl, Br, I, OH, CF₃, CHF₂, CH₂F, NO₂, CN, OCF₃, OCHF₂, -(C₁-C₆)-Alkylen-(R9), O-(C₁-C₆)-Alkylen-(R9), tertiär-Butyl, iso-Propylen-(R9), (C=O)-(C₁-C₆)-Alkylen-(R9), (C₁-C₆)-Alkylen-(R9), NH₂, NH(C₁-C₆)-Alkylen-(R9), N((C₁-C₆)-Alkylen-R9)₂, (C₁-C₆)-Alkylen-NH₂, (C₁-C₆)-Alkylen-NH(C₁-C₆)-Alkylen-(R9), (C₁-C₆)-Alkylen-N((C₁-C₆)-Alkylen-R9)_{2,}-O-(C₁-C₆)-Alkylen-NH₂, -O-(C₁-C₆)-Alkylen-NH(C₁-C₆)-Alkylen-(R9), -O-(C₁-C₆)-Alkylen-N((C₁-C₆)-Alkylen-R9)_{2,} -NH-(C₁-C₆)-Alkylen-NH₂, -NH-(C₁-C₆)-Alkylen-NH(C₁-C₆)-Alkylen-(R9), -NH-(C₁-C₆)-Alkylen-N((C₁-C₆)-Alkylen-R9)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
(C₆-C₁₀)-Aryl, -(C₁-C₆)-Alkylen-(C₆-C₁₀)-Aryl,,
wobei der Arylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅, (C₆-C₁₀)-Aryl, (C₃-C₇)-Cycloalkyl, 4 bis12-gliedriger Heterocyclus;
wobei der (C₆-C₁₀)-Arylrest, (C₃-C₇)-Cycloalkylrest, 4 bis12-gliedriger Heterocyclusrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
4 bis 12-gliedriger Heterocyclus, -(C₁-C₆)-Alkylen-4 bis12-gliedriger Heterocyclus,
wobei der Heterocyclylrest ein bis 3-fach substituiert sein kann mit F, Cl, Br, I, OH, CF₃, CHF₂, CH₂F, NO₂, CN, OCF₃, OCHF₂, O-(C₁-C₆)-Alkylen-(R9), (C₁-C₆)-Alkylen-(R9), NH₂, NH(C₁-C₆)-Alkylen-(R9), N((C₁-C₆)-Alkyl)₂, SO₂-(C₁-C₆)-Alkylen-(R9), SO₂-C₂H₂F₃, SO₂-CH₃, SO₂-NH₂, SO₂-NH(C₁-C₆)-Alkylen-(R9), SO₂-N((C₁-C₆)-Alkyl)₂, COOH, COO-(C₁-C₆)-Alkylen-(R9), CONH₂, CONH(C₁-C₆)-Alkylen-(R9), CON((C₁-C₆)-Alkyl)₂, SF_{5,}(C₆-C₁₀)-Aryl, (C₃-C₇)-Cycloalkyl, 4 bis12-gliedriger Heterocyclus;
wobei der (C₆-C₁₀)-Arylrest, (C₃-C₇)-Cycloalkylrest, 4 bis12-gliedriger Heterocyclusrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
R7, R8 unabhängig voneinander H, (C₁-C₃)-Alkyl, wobei der Alkylrest ein bis 3-fach mit Fluor substituiert sein kann, oder R7 und R8 bilden gemeinsam mit dem Kohlenstoffatom an das sie gebunden sind einen 3-7 gliedrigen Carbocyclus oder Heterocyclus;
R9 H, OH, OCH₃, OCF₃, CHF₂, CF₃;
R10 H, CONH₂;
sowie deren pharmazeutisch verträgliche Salze.

2. Verbindungen der Formel I, gemäß Anspruch 1, **dadurch gekennzeichnet, dass** darin bedeuten
R1 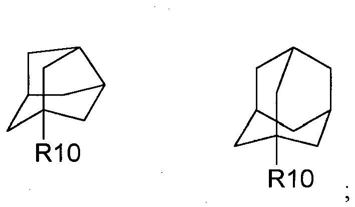
R2 H, (C₁-C₃)-Alkyl;
R3, R4, R5, R13 unabhängig voneinander
H, F, Cl, Br, I, OH, CF₃, CHF₂, CH₂F, NO₂, CN, OCF₃, OCHF₂, -(C₁-C₆)-Alkylen-(R9), O-(C₁-C₆)-Alkylen-(R9), tertiär-Butyl, iso-Propylen-(R9), (C=O)-(C₁-C₆)-Alkylen-(R9), (C₁-C₆)-Alkylen-(R9), NH₂, NH(C₁-C₆)-Alkylen-(R9), N((C₁-C₆)-Alkylen-R9)₂, (C₁-C₆)-Alkylen-NH₂, (C₁-C₆)-Alkylen-NH(C₁-C₆)-Alkylen-(R9), (C₁-C₆)-Alkylen-N((C₁-C₆)-Alkylen-R9)_{2,} -O-(C₁-C₆)-Alkylen-NH₂, -O-(C₁-C₆)-Alkylen-NH(C₁-C₆)-Alkylen-(R9), -O-(C₁-C₆)-Alkylen-N((C₁-C₆)-Alkylen-R9)₂,-NH-(C₁-C₆)-Alkylen-NH₂, -NH-(C₁-C₆)-Alkylen-NH(C₁-C₆)-Alkylen-(R9), -NH-(C₁-C₆)-Alkylen-N((C₁-C₆)-Alkylen-R9)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
(C₆-C₁₀)-Aryl, -(C₁-C₆)-Alkylen-(C₆-C₁₀)-Aryl,,
wobei der Arylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅; (C₆-C₁₀)-Aryl, (C₃-C₇)-Cycloalkyl, 4 bis12-gliedriger Heterocyclus;
wobei der (C₆-C₁₀)-Arylrest, (C₃-C₇)-Cycloalkylrest, 4 bis12-gliedriger Heterocyclusrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
4 bis 12-gliedriger Heterocyclus, -(C₁-C₆)-Alkylen-4 bis12-gliedriger Heterocyclus,
wobei der Heterocyclylrest ein bis 3-fach substituiert sein kann mit F, Cl, Br, I, OH, CF₃, CHF₂, CH₂F, NO₂, CN, OCF₃, OCHF₂, O-(C₁-C₆)-Alkylen-(R9), (C₁-C₆)-Alkylen-(R9), NH₂, NH(C₁-C₆)-Alkylen-(R9), N((C₁-C₆)-Alkyl)₂, SO₂-(C₁-C₆)-Alkylen-(R9), SO₂-C₂H₂F₃, SO₂-CH₃, SO₂-NH₂, SO₂-NH(C₁-C₆)-Alkylen-(R9), SO₂-N((C₁-C₆)-Alkyl)₂, COOH, COO-(C₁-C₆)-Alkylen-(R9), CONH₂, CONH(C₁-C₆)-Alkylen-(R9), CON((C₁-C₆)-Alkyl)₂, SF₅; (C₆-C₁₀)-Aryl, (C₃-C₇)-Cycloalkyl, 4 bis12-gliedriger Heterocyclus;
wobei der (C₆-C₁₀)-Arylrest, (C₃-C₇)-Cycloalkylrest, 4 bis12-gliedriger Heterocyclusrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
R7, R8 unabhängig voneinander H, (C₁-C₃)-Alkyl;
R9 H, OH, OCH₃, OCF₃, CHF₂, CF₃;
R10 H, CONH₂;
sowie deren pharmazeutisch verträgliche Salze.

3. Verbindungen der Formel I, gemäß Anspruch 1 oder 2, **dadurch gekennzeichnet, dass** darin bedeuten
R1 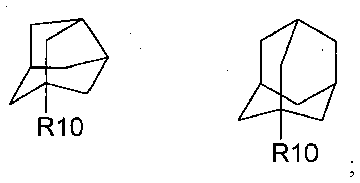
R2 H;
R3, R4, R5, R13 unabhängig voneinander H, F, Cl, Br, I, OH, CF₃, NO₂, CN, OCF₃, OCHF₂, -(C₁-C₆)-Alkylen, -O-(C₁-C₆)-Alkylen;
(C₆-C₁₀)-Aryl,
wobei der Arylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
4 bis 12-gliedriger Heterocyclus,
wobei der Heterocyclylrest ein bis 3-fach substituiert sein kann mit F, Cl, Br, I, OH, CF₃, CHF₂, CH₂F, NO₂, CN, OCF₃, OCHF₂, O-(C₁-C₆)-Alkylen-(R9), (C₁-C₆)-Alkylen-(R9), NH₂, NH(C₁-C₆)-Alkylen-(R9), N((C₁-C₆)-Alkyl)₂, SO₂-(C₁-C₆)-Alkylen-(R9), SO₂-C₂H₂F₃, SO₂-CH₃, SO₂-NH₂, SO₂-NH(C₁-C₆)-Alkylen-(R9), SO₂-N((C₁-C₆)-Alkyl)₂, COOH, COO-(C₁-C₆)-Alkylen-(R9), CONH₂, CONH(C₁-C₆)-Alkylen-(R9), CON((C₁-C₆)-Alkyl)₂, SF₅;
R7, R8 H, (C₁-C₃)-Alkyl;
R9 H, OH, OCH₃, OCF₃, CHF₂, CF₃;
R10 H, CONH₂;
sowie deren pharmazeutisch verträgliche Salze.

4. Verbindungen der Formel I, gemäß einem oder mehreren der Ansprüche 1 bis 3, **dadurch gekennzeichnet, dass** darin bedeuten
R1 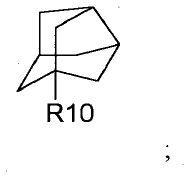
R2 H;
R3, R4, R5 unabhängig voneinander
H, F, Cl, Br, I, OH, CF₃, NO₂, CN, OCF₃, OCHF₂, -(C₁-C₆)-Alkylen, -O-(C₁-C₆)-Alkylen;
Phenyl,
wobei der Phenylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
Piperidinyl,
wobei der Piperidinyllrest ein bis 3-fach substituiert sein kann mit F, Cl, Br, I, OH, CF₃, CHF₂, CH₂F, NO₂, CN, OCF₃, OCHF₂, O-(C₁-C₆)-Alkylen-(R9), (C₁-C₆)-Alkylen-(R9), NH₂, NH(C₁-C₆)-Alkylen-(R9), N((C₁-C₆)-Alkyl)₂, SO₂-(C₁-C₆)-Alkylen-(R9), SO₂-C₂H₂F₃, SO₂-CH₃, SO₂-NH₂, SO₂-NH(C₁-C₆)-Alkylen-(R9), SO₂-N((C₁-C₆)-Alkyl)₂, COOH, COO-(C₁-C₆)-Alkylen-(R9), CONH₂, CONH(C₁-C₆)-Alkylen-(R9), CON((C₁-C₆)-Alkyl)₂, SF₅;
R13 F, Cl, Br, I, OH, CF₃, NO₂, CN, OCF₃, OCHF₂, -(C₁-C₆)-Alkylen, -O-(C₁-C₆)-Alkylen;
Phenyl,
wobei der Phenylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
Piperidinyl,
wobei der Piperidinyllrest ein bis 3-fach substituiert sein kann mit F, Cl, Br, I, OH, CF₃, CHF₂, CH₂F, NO₂, CN, OCF₃, OCHF₂, O-(C₁-C₆)-Alkylen-(R9), (C₁-C₆)-Alkylen-(R9), NH₂, NH(C₁-C₆)-Alkylen-(R9), N((C₁-C₆)-Alkyl)₂, SO₂-(C₁-C₆)-Alkylen-(R9), SO₂-C₂H₂F₃, SO₂-CH₃, SO₂-NH₂, SO₂-NH(C₁-C₆)-Alkylen-(R9), SO₂-N((C₁-C₆)-Alkyl)₂, COOH, COO-(C₁-C₆)-Alkylen-(R9), CONH₂, CONH(C₁-C₆)-Alkylen-(R9), CON((C₁-C₆)-Alkyl)₂, SF₅;
R7, R8 H, (C₁-C₃)-Alkyl;
R9 H, OH, OCH₃, OCF₃, CHF₂, CF₃;
R10 H, CONH₂;
sowie deren pharmazeutisch verträgliche Salze.

5. Verbindungen der Formel I, gemäß einem oder mehreren der Ansprüche 1 bis 4, **dadurch gekennzeichnet, dass** darin der Rest R1 wie in Formel Ia und Ib gezeigt verknüpft ist 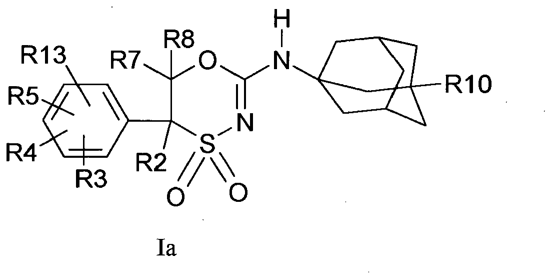 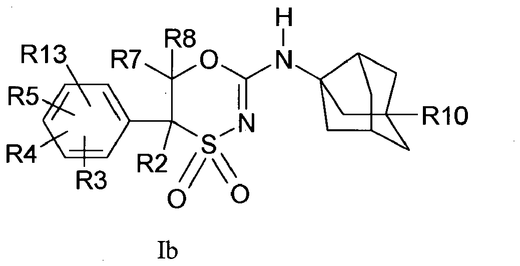 sowie deren pharmazeutisch verträgliche Salze.

6. Verbindungen der Formel I, gemäß einem oder mehreren der Ansprüche 1 bis 5, zur Anwendung als Arzneimittel.

7. Arzneimittel enthaltend Verbindungen der Formel I, gemäß einem oder mehreren der Ansprüche 1 bis 5.

8. Arzneimittel enthaltend Verbindungen der Formel I, gemäß einem oder mehreren der Ansprüche 1 bis 5 und mindestens einen weiteren Wirkstoff.

9. Arzneimittel, gemäß Anspruch 8, **dadurch gekennzeichnet, dass** es als weiteren Wirkstoff eine oder mehrere Antidiabetika, hypoglykämischen Wirkstoffe, HMGCoA-Reduktase Inhibitoren, Cholesterinresorptionsinhibitoren, PPAR gamma Agonisten, PPAR alpha Agonisten, PPAR alpha/gamma Agonisten, PPAR delta Agonisten, Fibrate, MTP-Inhibitoren, Gallensäureresorptionsinhibitoren, CETP-Inhibitoren, polymere Gallensäureadsorber, LDL-Rezeptorinducer, ACAT-Inhibitoren, Antioxidantien, Lipoprotein-Lipase Inhibitoren, ATP-Citrat-Lyase Inhibitoren, Squalen Synthetase Inhibitoren, Lipoprotein(a) Antagonisten, HM74A Rezeptor Agonisten, Lipase Inhibitoren, Insuline, Sulfonylharnstoffe, Biguanide, Meglitinide, Thiazolidindione, α-Glukosidase-Inhibitoren, auf den ATP-abhängigen Kaliumkanal der Betazellen wirkende Wirkstoffe, Glykogen Phosphorylase Inhibitoren, Glukagon-Rezeptor-Antagonisten, Aktivatoren der Glukokinase, Inhibitoren der Glukoneogenese, Inhibitoren der Fructose-1,6-biphosphatase, Modulatoren des Glukosetransporters-4, Inhibitoren der Glutamin-Fructose-6-Phosphat-Amidotransferase, Inhibitoren der Dipeptidylpeptidase-IV, Hemmstoffe der 11-beta-Hydroxysteroid-Dehydrogenase-1, Inhibitoren der Protein-Tyrosin-Phosphatase-1B, Modulatoren des natrium-abhängigen Glukosetransporters 1 oder 2, Modulatoren des GPR40, Inhibitoren der hormon-sensitiven Lipase, Hemmstoffe der Acetyl-CoA Carboxylase, Inhibitoren der Phosphoenolpyruvatcarboxykinase, Inhibitoren der Glykogen Synthase Kinase-3 beta, Inhibitoren der Protein Kinase C beta, Endothelin-A-Rezeptor Antagonisten, Inhibitoren der I kappaB Kinase, Modulatoren des Glukocorticoidrezeptors, CART-Agonisten, NPY-Agonisten, MC4-Agonisten, Orexin-Agonisten, H3-Agonisten, TNF-Agonisten, CRF-Agonisten, CRF BP-Antagonisten, Urocortin-Agonisten, β3-Agonisten, , CB1-Rezeptor Antagonisten ,MSH (Melanocyt-stimulierendes Hormon)-Agonisten, CCK-Agonisten, Serotonin-Wiederaufnahme-Inhibitoren, gemischte Sertonin- und noradrenerge Verbindungen, 5HT-Agonisten, Bombesin-Agonisten, Galanin-Antagonisten, Wachstumshormone, Wachstumshormon freisetzende Verbindungen, TRH-Agonisten, entkoppelnde Protein 2- oder 3-Modulatoren, Leptinagonisten, DA-Agonisten , Lipase/Amylase-Inhibitoren, PPAR-Modulatoren, RXR-Modulatoren oder TR-β-Agonisten oder Amphetamine enthält.

10. Verfahren zur Herstellung eines Arzneimittels enthaltend die Verbindungen der Formel I, gemäß einem oder mehreren der Ansprüche 1 bis 5, **dadurch gekennzeichnet, daß** der Wirkstoff mit einem pharmazeutisch geeigneten Träger vermischt wird und diese Mischung in eine für die Verabreichung geeignete Form gebracht wird.

11. Verwendung der Verbindungen der Formel I, gemäß einem oder mehreren der Ansprüche 1 bis 5 zur Herstellung eines Medikaments zur Behandlung von Hyperglykämie.

12. Verwendung der Verbindungen der Formel I, gemäß einem oder mehreren der Ansprüche 1 bis 5 zur Herstellung eines Medikaments zur Behandlung des Diabetes.

13. Verwendung der Verbindungen der Formel I, gemäß einem oder mehreren der Ansprüche 1 bis 5 zur Herstellung eines Medikaments zur Behandlung von Insulin Resistenz.

14. Set (Kit) bestehend aus getrennten Packungen von
a) einer wirksamen Menge einer Verbindung der Formel I, gemäß einem oder mehreren der Ansprüche 1 bis 5 und
b) einer wirksamen Menge eines weiteren Arzneimittelswirkstoffs.

## Claims

1. Compounds of the formula I 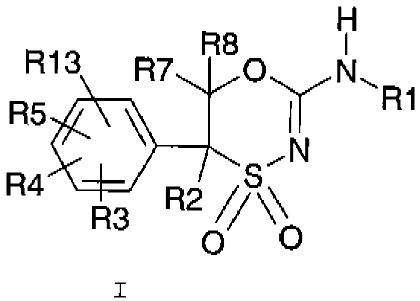 in which
R1 is 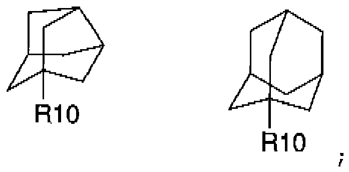
R2 is H, F, (C₁-C₃)-alkyl where the alkyl radical may be mono- to trisubstituted by fluorine;
R3, R4, R5, R13 are each independently H, F, Cl, Br, I, OH, CF₃, CHF₂, CH₂F, NO₂, CN, OCF₃, OCHF₂, - (C₁-C₆) - alkylene-(R9), O-(C₁-C₆)-alkylene-(R9), tert-butyl, isopropylene-(R9), (C=O) - (C₁-C₆)-alkylene-(R9), (C₁-C₆)-alkylene-(R9), NH₂, NH (C₁-C₆)-alkylene-(R9), N((C₁-C₆)-alkylene-R9)₂, (C₁-C₆)-alkylene-NH₂, (C₁-C₆) -alkylene-NH (C₁-C₆) -alkylene- (R9), (C₁-C₆) -alkylene-N ((C₁-C₆) -alkylene-R9)₂, -O- (C₁-C₆)-alkylene-NH₂, -O- (C₁-C₆) -alkylene-NH(C₁-C₆) -alkylene- (R9), -0- (C₁-C₆) -alkylene-N((C₁-C₆)-alkylene-R)₂, -NH-(C₁-C₆)-alkylene-NH₂,-NH-(C₁-C₆)-alkylene-NH(C₁-C₆)-alkylene-(R9), NH- (C₁-C₆) -alkylene-N( ( (C₁-C₆) -alkylene-R9)₂, SO₂-CH₃, SO₃-NH₂, SO₂-NH(C₁-C₆)-alkyl, SO₂-N((C₁-C₆) -alkyl)₂, COOH, COO- (C₁-C₆) -alkyl, CONH₂, CONH(C₁-C₆)-alkyl, CON((C₁-C₆)-alkyl)₂, SF₅;
(C₆-C₁₀) -aryl , - (C₁-C₆) -alkylene- (C₆-C₁₀) -aryl,
where the aryl radical may be mono- to trisubstituted by F, Cl, Br, I, OH, CF₃, CHF₂, CH₂F, NO₂, CN, OCF₃, OCHF₂, O-(C₁-C₆)-alkyl, (C₁-C₆)-alkyl, NH₂, NH(C₁-C₆)-alkyl, N((C₁-C₆)-alkyl)₂, SO₂-CH₃, SO₂-NH₂, SO₂-NH(C₁-C₆)-alkyl, SO₂-N((C₁-C₆)-alkyl)₃, COOH, COO-(C₁-C₆)-alkyl, CONH₂, CONH(C₁-C₆)-alkyl, CON((C₁-C₆)-alkyl)₃, SF₅; (C₆-C₁₀)-aryl, (C₃-C₇)-cycloalkyl, 4- to 12-membered heterocycle;
where the (C₆-C₁₀)-aryl radical, (C₃-C₇)-cycloalkyl radical, 4- to 12-membered heterocycle radical may be mono- to trisubstituted by F, Cl, Br, I, OH, CF₃, CHF₂, CH₂F, NO₂, CN, OCF₃, OCHF₂, O-(C₁-C₆)-alkyl, (C₁-C₆)-alkyl, NH₂, NH(C₁-C₆)-alkyl, N ((C₁-C₆) -alkyl)₂, SO₂-CH₃, SO₂-NH₂, SO₂-NH(C₁-C₆)-alkyl, SO₂-N((C₁-C₆)-alkyl)₂, COOH, COO-(C₁-C₆)-alkyl, CONH₂, CONH(C₁-C₆)-alkyl, CON((C₁-C₆)-alkyl)₂, SF₅;
4- to 12-membered heterocycle, -(C₁-C₆)-alkylene-4-to -12-membered heterocycle,
where the heterocyclyl radical may be mono- to trisubstituted by F, Cl, Br, I, OH, CF₃, CHF₂, CH₂F, NO₂, CN, OCF₃, OCHF₂, O-(C₁-C₆)-alkylene-(R9), (C₁-C₆)-alkylene-(R9), NH₂, NH(C₁-C₆)-alkylene-(R9), N((C₁-C₆)-alkyl)₂, SO₂-(C₁-C₆)-alkylene-(R9), SO₂-C₂H₂F₃, SO₂-CH₃, SO₂-NH₂, SO₂-NH(C₁-C₆)-alkylene-(R9), SO₂-N((C₁-C₆)-alkyl)₂, COOH, COO- (C₁-C₆) -alkylene- (R9), CONH₂, CONH(C₁-C₆) -alkylene- (R9), CON ((C₁-C₆)-alkyl)₂, SF₅, (C₆-C₁₀)-aryl, (C₃-C₇)-cycloalkyl, 4- to 12-membered heterocycle;
where the (C₆-C₁₀)-aryl radical, (C₃-C₇)-cycloalkyl radical, 4- to 12-membered heterocycle radical may be mono- to trisubstituted by F, Cl, Br, I, OH, CF₃, CHF₂, CH₂F, NO₂, CN, OCF₃, OCHF₂, O-(C₁-C₆)-alkyl, (C₁-C₆)-alkyl, NH₂, NH(C₁-C₆)-alkyl, N((C₁-C₆)-alkyl)₂, SO₂-CH₃, SO₂-NH₂, SO₂-NH(C₁-C₆)-alkyl, SO₂-N((C₁-C₆)-alkyl)₂, COOH, COO-(C₁-C₆)-alkyl, CONH₂, CONH(C₁-C₆)-alkyl, CON((C₁-C₆)-alkyl)₂, SF₅;
R7, R8 are each independently H, (C₁-C₃)-alkyl, where the alkyl radical may be mono- to trisubstituted by fluorine, or R7 and R8 together with the carbon atom to which they are bonded form a 3-7-membered carbocycle or heterocycle;
R9 is H, OH, OCH₃, OCF₃, CHF₂, CF₃;
R10 is H, CONH₂;
and pharmaceutically compatible salts thereof.

2. Compounds of the formula I according to Claim 1, **characterized in that**
R1 is 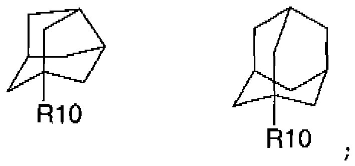
R2 is H, (C₁-C₃)-alkyl;
R3, R4, R5, R13 are each independently H, F, Cl, Br, I, OH, CF₃, CHF₂, CH₂F, NO₂, CN, OCF₃, OCHF₂, -(C₁-C₆)-alkylene-(R9), O-C₁-C₆)-alkylene-(R9), tert-butyl, isopropylene-(R9), (C=O)-(C₁-C₆)-alkylene-(R9), (C₁-C₆)-alkylene-(R9), NH₂, NH(C₁-C₆)-alkylene-(R9), N((C₁-C₆)-alkylene-R9)₂, (C₁-C₆)-alkylene-NH₂, (C₁-C₆)-alkylene-NH (C₁-C₆)-alkylene- (R9), (C₁-C₆)-alkylene-N ((C₁-C₆)-alkylene-R9)₂, -O-(C₁-C₆)-alkylene-NH₂, -O-(C₁-C₆)-alkylene-NH(C₁-C₆)-alkylene- (R9), -0- (C₁-C₆)-alkylene-N((C₁-C₆)-alkylene-R9)₂, -NH- (C₁-C₆)-alkylene-NH₂,-NH- (C₁-C₆)-alkylene-NH(C₁-C₆)-alkylene- (R9),-NH- (C₁-C₆)-alkylene-N((C₁-C₆)-alkylene-R9)₂, SO₂-CH₃, SO₂-NH₂, SO₂-NH(C₁-C₆)-alkyl, SO₂-N(C₁-C₆)-alkyl)₂, COOH, COO-(C₁-C₆)-alkyl, CONH₂, CONH(C₁-C₆)-alkyl, CON((C₁-C₆)-alkyl)₂, SF₅;
(C₆-C₁₀)-aryl, - (C₁-C₆)-alkylene- (C₆-C₁₀)-aryl,
where the aryl radical may be mono- to trisubstituted by F, Cl, Br, I, OH, CF₃, CHF₂, CH₂F, NO₂, CN, OCF₃, OCHF₂, O-(C₁-C₆)-alkyl, (C₁-C₆)-alkyl, NH₂, NH(C₁-C_{E})-alkyl, N((C₁-C₆)-alkyl)₂, SO₂-CH₃, SO₂-NH₂, SO₂-NH(C₁-C₆)-alkyl, SO₂-N((C₁-C₆)-alkyl)₂, COOH, COO-(C₁-C₆)-alkyl, CONH₂, CONH(C₁-C₆-alkyl, CON((C₁-C₆)-alkyl)₂, SF₅, (C₆-C₁₀)-aryl, (C₃-C₇)-cycloalkyl, 4- to 12-membered heterocycle;
where the (C₆-C₁₀)-aryl radical, (C₃-C₇)-cycloalkyl radical, 4- to 12-membered heterocycle radical may be mono- to trisubstituted by F, Cl, Br, I, OH, CF₃, CHF₂, CH₃F, NO₂, CN, OCF₃, OCHF₂, O-(C₁-C₆)-alkyl, (C₁-C₆)-alkyl, NH₂, NH(C₁-C₆)-alkyl, N((C₁-C₆)-alkyl)₂, SO₂-CH₃, SO₂-NH₂, SO₃-NH(C₁-C₆)-alkyl, SO₂-N((C₁-C₆)-alkyl)₂, COOH, COO-(C₁-C₆)-alkyl, CONH₂, CONH(C₁-C₆)-alkyl, CON((C₁-C₆)-alkyl)₂, SF₅;
4- to 12-membered heterocycle, -(C₁-C₆)-alkylene-4-to -12-membered heterocycle,
where the heterocyclyl radical may be mono- to trisubstituted by F, Cl, Br, I, OH, CF₃, CHF₂, CH₂F, NO₂, CN, OCF₃, OCHF₂, O-(C₁-C₆)-alkylene-(R9), (C₁-C₆)-alkylene-(R9), NH₂, NH(C₁-C₆)-alkylene- (R9), N((C₁-C₆)-alkyl)₂, SO₂-(C₁-C₆)-alkylene-(R9), SO₂-C₂H₂F₃, SO₂-CH₃, SO₂-NH₂, SO₂-NH(C₁-C₆)-alkylene-(R9), SO₂-N(C₁-C₆)-alkyl)₂, COOH, COO-(C₁-C₆)-alkylene-(R9), CONH₂, CONH(C₁-C₆)-alkylene-(R9), CON((C₁-C₆)-alkyl)₂, SF₅, (C₆-C₁₀)-aryl, (C₃-C₇)-cycloalkyl, 4- to 12-membered heterocycle;
where the (C₆-C₁₀)-aryl radical, (C₃-C₇)-cycloalkyl radical, 4- to 12-membered heterocycle radical may be mono- to trisubstituted by F, Cl, Br, I, OH, CF₃, CHF₂, CH₂F, NO₂, CN, OCF₃, OCF₂, O-(C₁-C₆)-alkyl, (C₁-C₆)-alkyl, NH₂, NH(C₁-C₆)-alkyl, N((C₁-C₆)-alkyl)₂, SO₂-CH₃, SO₂-NH₂, SO₂-NH(C₁-C₆)-alkyl, SO₂-N((C₁-C₆)-alkyl)₃, COOH, COO-(C₁-C₆)-alkyl, CONH₂, CONH(C₁-C₆)-alkyl, CON((C₁-C₆)-alkyl)₂, SF₅;
R7, R8 are each independently H, (C₁-C₃)-alkyl;
R9 is H, OH, OCH₃, OCF₃, CHF₂, CF₃;
R10 is H, CONH₂;
and pharmaceutically compatible salts thereof.

3. Compounds of the formula I according to Claim 1 or 2, **characterized in that** is
R1 is 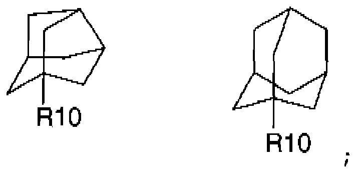
R2 is H;
R3, R4, R5, R13 are each independently
H, F, Cl, Br, I, OH, CF₃, NO₂, CN, OCF₃, OCHF₂,-(C₁-C₆)-alkylene, -O-(C₁-C₆)-alkylene;
(C₆-C₁₀)-aryl,
where the aryl radical may be mono- to trisubstituted by F, Cl, Br, I, OH, CF₃, CHF₂, CH₂F, NO₂, CN, OCF₃, OCHF₂, O-(C₁-C₆)-alkyl, (C₁-C₆)-alkyl, NH₂, NH(C₁-C₆)-alkyl, N((C₁-C₆)-alkyl)₂, SO₂-CH₃, SO₂-NH₂, SO₂-NH(C₁-C₆)-alkyl, SO₂-N((C₁-C₆)-alkyl)₂, COOH, COO-(C₁-C₆)-alkyl, CONH₂, CONH(C₁-C₆)-alkyl, CON((C₂-C₆)-alkyl)₂, SF₅;
4- to 12-membered heterocycle,
where the heterocyclyl radical may be mono- to trisubstituted by F, Cl, Br, I, OH, CF₃, CHF₂, CH₂F, NO₂, CN, OCF₃, OCHF₂, O-(C₁-C₆)-alkylene-(R9), (C₁-C₆)-alkylene- (R9), NH₂, NH(C₁-C₆)-alkylene- (R9), N((C₁-C₆)-alkyl)₂, SO₂-(C₁-C₆)-alkylene-(R9), SO₂-C₂H₂F₃, SO₂-CH₃, SO₂-NH₂, SO₂-NH(C₁-C₆)-alkylene-(R9), SO₃-N((C₁-C₆)-alkyl)₂, COOH, COO-(C₁-C₆)-alkylene-(R9), CONH₂, CONH(C₁-C₆)-alkylene-(R9), CON((C₁-C₆)-alkyl)₂, SF₅;
R7, R8 is H, (C₁-C₃)-alkyl;
R9 is H, OH, OCH₃, OCF₃, CHF₂, CF₃;
R10 is H, CONH₂;
and pharmaceutically compatible salts thereof.

4. Compounds of the formula I according to one or more of Claims 1 to 3, **characterized in that**
R1 is 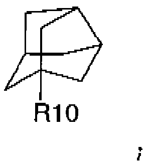
R2 is H;
R3, R4, R5 are each independently
H, F, Cl, Br, I, OH, CF₃, NO₂, CN, OCF₃, OCHF₂,-(C₁-C₆)-alkylene, -O-(C₁-C₆)-alkylene;
phenol,
where the phenyl radical may be mono- to trisubstituted by F, Cl, Br, I, OH, CF₃, CHF₂, CH₂F, NO₂, CN, OCF₃, OCHF₂, O-(C₁-C₆)-alkyl, (C₁-C₆)-alkyl, NH₂, NH(C₁-C₆)-alkyl, N((C₁-C₆)-alkyl)₂, SO₂-CH₃, SO₂-NH₂, SO₂-NH(C₁-C₆)-alkyl, SO₂-N((C₁-C₆)-alkyl)₂, COOH, COO-(C₁-C₆)-alkyl, CONH₂, CONH(C₁-C₆))-alkyl, CON((C₁-C₆)-alkyl)₂, SF₅; piperidinyl,
where the piperidinyl radical may be mono- to trisubstituted by F, Cl, Br, I, OH, CF₃, CHF₂, CH₂F, NO₂, CN, OCF₃, OCHF₂, O-(C₁-C₆) -alkylene- (R9), (C₁-C₆)-alkylene- (R9), NH₂, NH(C₁-C₆)-alkylene-(R9), N((C₁-C₆)-alkyl)₂, SO₂-(C₁-C₆)-alkylene-(R9), SO₂-C₂H₂F₃, SO₂-CH₃, SO₂-NH₃, SO₂-NH(C₁-C₆)-alkylene-(R9), SO₂-N((C₁-C₆)-alkyl)₂, COOH, COO-(C₁-C₆)-alkylene- (R9), CONH₂, CONH(C₁-C₆)-alkylene-(R9), CON((C₁-C₆)-alkyl)₂, SF₅;
R13 is F, Cl, Br, I, OH, CF₃, NO₂, CN, OCF₃, OCHF₂,-(C₁-C₆)-alkylene, -O-(C₁-C₆)-alkylene; phenyl,
where the phenyl radical may be mono- to trisubstituted by F, Cl, Br, I, OH, CF₃, CHF₂, CH₂F, NO₂, CN, OCF₃, OCHF₂, O-(C₁-C₆)-alkyl, (C₁-C₆)-alkyl, NH₂, NH(C₁-C₆)-alkyl, N((C₁-C₆)-alkyl)₂, SO₂-CH₃, SO₂-NH₃, SO₂-NH(C₁-C₆)-alkyl, SO₂-N((C₁-C₆)-alkyl)₂, COOH, COO-(C₁-C₆)-alkyl, CONH₂, CONH(C₁-C₆)-alkyl, CON((C₁-C₆)-alkyl)₂, SF₅;
piperidinyl,
where the piperidinyl radical may be mono- to trisubstituted by F, Cl, Br, I, OH, CF₃, CHF₂, CH₂F, NO₂, CN, OCF₃, OCHF₂, O-(C₁-C₆)-alkylene-(R9), (C₁-C₆-alkylene-(R9), NH₂, NH(C₁-C₆)-alkylene-(R9), N((C₁-C₆)-alkyl)₂, SO₂- (C₁-C₆)-alkylene- (R9), SO₂-C₂H₂F₃, SO₂-CH₃, SO₂-NH₂, SO₂-NH(C₁-C₆)-alkylene-(R9), SO₂-N((C₁-C₆)-alkyl)₂, COOH, COO-(C₁-C₆)-alkylene-(R9), CONH₂, CONH(C₁-C₆)-alkylene-(R9), CON((C₁-C₆)-alkyl)₂, SF₅;
R7, R8 is H, (C₁-C₃)-alkyl;
R9 is H, OH, OCH₃, OCF₃, CHF₂, CF₃;
R10 is H, CONH₂;
and pharmaceutically compatible salts thereof.

5. Compounds of the formula I according to one or more of Claims 1 to 4, **characterized in that** the R1 radical therein is bonded as shown in formula Ia and Ib 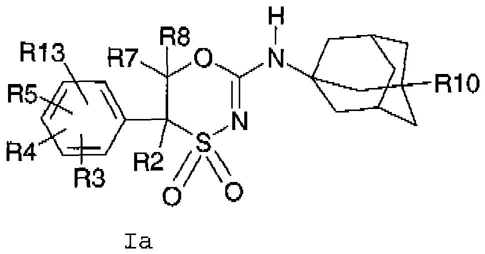 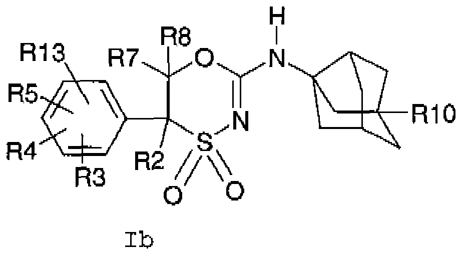 and pharmaceutically compatible salts thereof.

6. Compounds of the formula I according to one or more of Claims 1 to 5 for use as medicaments.

7. Medicament comprising compounds of the formula I according to one or more of Claims 1 to 5.

8. Medicament comprising compounds of the formula I according to one or more of Claims 1 to 5 and at least one further active ingredient.

9. Medicament according to Claim 8, **characterized in that** it comprises, as a further active ingredient, one or more antidiabetics, active hypoglycemic ingredients, HMG-CoA reductase inhibitors, cholesterol absorption inhibitors, PPAR gamma agonists, PPAR alpha agonists, PPAR alpha/gamma agonists, PPAR delta agonists, fibrates, MTP inhibitors, bile acid absorption inhibitors, CETP inhibitors, polymeric bile acid adsorbers, LDL receptor inducers, ACAT inhibitors, antioxidants, lipoprotein lipase inhibitors, ATP citrate lyase inhibitors, squalene synthetase inhibitors, lipoprotein(a) antagonists, HM74A receptor agonists, lipase inhibitors, insulins, sulfonylureas, biguanides, meglitinides, thiazolidinediones, α-glucosidase inhibitors, active ingredients which act on the ATP-dependent potassium channel of the beta cells, glycogen phosphorylase inhibitors, glucagon receptor antagonists, activators of glucokinase, inhibitors of gluconeogenesis, inhibitors of fructose 1,6-biphosphatase, modulators of glucose transporter 4, inhibitors of glutamine:fructose-6-phosphate amidotransferase, inhibitors of dipeptidylpeptidase IV, inhibitors of 11-beta-hydroxysteroid dehydrogenase 1, inhibitors of protein tyrosine phosphatase 1B, modulators of the sodium-dependent glucose transporter 1 or 2, GPR40 modulators, inhibitors of hormone-sensitive lipase, inhibitors of acetyl-CoA carboxylase, inhibitors of phosphoenolpyruvate carboxykinase, inhibitors of glycogen synthase kinase-3 beta, inhibitors of protein kinase C beta, endothelin-A receptor antagonists, inhibitors of I kappaB kinase, modulators of the glucocorticoid receptor, CART agonists, NPY agonists, MC4 agonists, orexin agonists, H3 agonists, TNF agonists, CRF agonists, CRF BP antagonists, urocortin agonists, β3 agonists, CB1 receptor antagonists, MSH (melanocyte-stimulating hormone) agonists, CCK agonists, serotonin reuptake inhibitors, mixed serotoninergic and noradrenergic compounds, 5HT agonists, bombesin agonists, galanin antagonists, growth hormones, growth hormone-releasing compounds, TRH agonists, decoupling protein 2 or 3 modulators, leptin agonists, DA agonists, lipase/amylase inhibitors, PPAR modulators, RXR modulators or TR β agonists or amphetamines.

10. Process for producing a medicament comprising the compounds of the formula I according to one or more of Claims 1 to 5, **characterized in that** the active ingredient is mixed with a pharmaceutically suitable carrier and this mixture is converted to a form suitable for administration.

11. Use of the compounds of the formula I according to one or more of Claims 1 to 5 for producing a medicament for treatment of hyperglycemia.

12. Use of the compounds of the formula I according to one or more of Claims 1 to 5 for producing a medicament for treatment of diabetes.

13. Use of the compounds of the formula I according to one or more of Claims 1 to 5 for producing a medicament for treatment of insulin resistance.

14. Set (kit) consisting of separate packages of
a) an effective amount of a compound of the formula I according to one or more of Claims 1 to 5 and
b) an effective amount of a further active medicament ingredient.

## Revendications

1. Composés de formule I 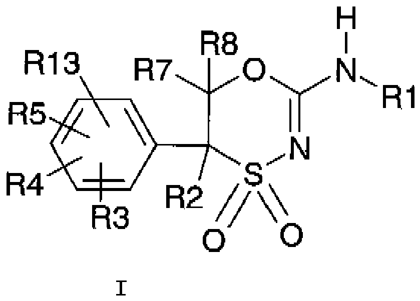 dans laquelle
R1 signifie 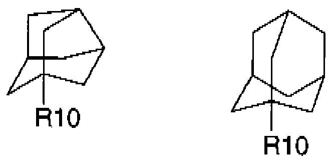
R2 signifie H, F, alkyle en (C₁-C₃), le radical alkyle pouvant être substitué une à 3 fois avec fluor ;
R3, R4, R5, R13 signifient indépendamment les uns des autres
H, F, Cl, Br, I, OH, CF₃, CHF₂, CH₂F, NO₂, CN, OCF₃, OCHF₂, alkylène en (C₁-C₆)-(R9), O-alkylène en (C₁-C₆)-(R9), tert.-butyle, iso-propylène-(R9), (C=O)-alkylène en (C₁-C₆)-(R9), alkylène en (C₁-C₆)-(R9), NH₂, NH-alkylène en (C₁-C₆)-(R9), N(alkylène en (C₁-C₆)-R9)₂, alkylène en (C₁-C₆)-NH₂, alkylène en (C₁-C₆)-NH-alkylène en (C₁-C₆)-(R9), alkylène en (C₁-C₇)-N(alkylène en (C₁-C₆)-R9)₂, -O-alkylène en (C₁-C₆)-NH₂, -O-alkylène en (C₁-C₆)-NH-alkylène en (C₁-C₆)-(R9), -O-alkylène en (C₁-C₆)-N(alkylène en (C₁-C₆)-R9)₂, -NH-alkylène en (C₁-C₆)-NH₂, -NH-alkylène en (C₁-C₆)-NH-alkylène en (C₁-C₆)-(R9), -NH-alkylène en (C₁-C₆)-N(alkylène en (C₁-C₆)-R9)₂, SO₂-CH₃, SO₂-NH₂, SO₂-NH-alkyle en (C₁-C₆), SO₂-N(alkyle en (C₁-C₆))₂, COOH, COO-alkyle en (C₁-C₆), CONH₂, CONH-alkyle en (C₂-C₆), CON(alkyle en (C₁-C₆))₂, SF₅; aryle en (C₆-C₁₀), alkylène en (C₂-C₆)-aryle en (C₆-C₁₀),
le radical aryle pouvant être substitué une à 3 fois avec F, Cl, Br, **I**, OH, CF₃, CHF₂, CH₂F, NO₂, CN, OCF₃, OCHF₂, O-alkyle en (C₁-C₆), alkyle en (C₁-C₆), NH₂, NH-alkyle en (C₁-C₆), N(alkyle en (C₁-C₆))₂, SO₂-CH₃, SO₂-NH₂, SO₂-NH-alkyle en (C₁-C₆), SO₂-N(alkyle en (C₁-C₆))₂, COOH, COO-alkyle en (C₁-C₆), CONH₂, CONH-alkyle en (C₁-C₆), CON(alkyle en (C₁-C₆))₂, SF₅, aryle en (C₆-C₁₀), cycloalkyle en (C₃-C₇), hétérocycle de 4 à 12 éléments ;
le radical aryle en (C₆-C₁₀), le radical cycloalkyle en (C₃-C₇), l'hétérocycle de 4 à 12 éléments pouvant être substitués une à 3 fois avec F, Cl, Br, I, OH, CF₃, CHF₂, CH₂F, NO₂, CN, OCF₃, OCHF₂, O-alkyle en (C₁-C₆), alkyle en (C₁-C₆), NH₂, NH-alkyle en (C₁-C₆), N(alkyle en (C₁-C₆))₂, SO₂-CH₃, SO₂-NH₂, SO₂-NH-alkyle en (C₁-C₆), SO₂-N(alkyle en (C₁-C₆))₂, COOH, COO-alkyle en (C₁-C₆), CONH₂, CONH-alkyle en (C₁-C₆), CON(alkyle en (C₁-C₆))₂, SF₅;
hétérocycle de 4 à 12 éléments, alkylène en (C₁-C₆)-hétérocycle de 4 à 12 éléments,
le radical hétérocyclyle pouvant être substitué une à 3 fois avec F, Cl, Br, I, OH, CF₃, CHF₂, CH₂F, NO₂, CN, OCF₃, OCHF₂, O-alkylène en (C₁-C₆)-(R9), alkylène en (C₁-C₆)-(R9), NH₂, NH-alkylène en (C₁-C₆)-(R9), N(alkyle en (C₁-C₆))₂, SO₂-alkylène en (C₁-C₆)-(R9), SO₂-C₂H₂F₃, SO₂-CH₃, SO₂-NH₂, SO₂-NH-alkylène en (C₁-C₆)-(R9), SO₂-N(alkyle en (C₁-C₆))₂, COOH, COO-alkylène en (C₁-C₆)-(R9), CONH₂, CONH-alkylène en (C₁-C₆)-(R9), CON(alkyle en (Cᵢ-Cs))₂, SF₅, aryle en (C₆-C₁₀), cycloalkyle en (C₃-C₇), hétérocycle de 4 à 12 éléments ;
le radical aryle en (C₆-C₁₀), le radical cycloalkyle en (C₃-C₇), l'hétérocycle de 4 à 12 éléments pouvant être substitués une à 3 fois avec F, Cl, Br, I, OH, CF₃, CHF₂, CH₂F, NO₂, CN, OCF₃, OCHF₂, O-alkyle en (C₁-C₆), alkyle en (C₁-C₆), NH₂, NH-alkyle en (C₁-C₆), N(alkyle en (C₁-C₆))₂, SO₂-CH₃, SO₂-NH₂, SO₂-NH-alkyle en (C₁-C₆), SO₂-N(alkyle en (C₁-C₆))₂, COOH, COO-alkyle en (C₁-C₆), CONH₂, CONH-alkyle en (C₁-C₆), CON(alkyle en (C₁-C₆))₂, SF₅;
R7, R8 signifient indépendamment l'un de l'autre H, alkyle en (C₁-C₃), le radical alkyle pouvant être substitué une à 3 fois avec fluor, ou R7 et R8 forment ensemble avec l'atome de carbone auquel ils sont reliés un carbocycle ou hétérocycle de 3 à 7 éléments;
R9 signifie H, OH, OCH₃**,** OCH₃, CHF₂, CF₃;
R10 signifie H, CONH₂;
ainsi que leurs sels pharmaceutiquement compatibles.

2. Composés de formule **I** selon la revendication 1, **caractérisés en ce que**
R1 signifie; 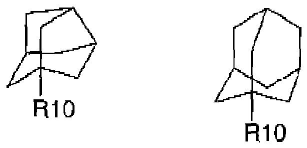
R2 signifie H, alkyle en (C₁-C₃);
R3, R4, R5, R13 signifient indépendamment les uns des autres
H, F, Cl, B, I, OH, CF₃, CHF₂, CH₂F, NO₂, CN, OCF₃, OCHF₂, alkylène en (C₁-C₆)-(R9), O-alkylène en (C₁-C₆)-(R9), tert.-butyle, iso-propylène-(R9), (C=O)-alkylène en (C₁-C₆)-(R9), alkylène en (C₁-C₆)-(R9), NH₂, NH-alkylène en (C₁-C₆)-(R9), N(alkylène en (C₁-C₆)-R9)₂, alkylène en (C₁-C₆)-NH₂, alkylène en (C₁-C₆)-NH-alkylène en (C₁-C₆)-(R9), alkylène en (C₁-C₆)-N(alkylène en (C₁-C₆)-R9)₂, -O-alkylène en (C₁-C₆)-NH₂, -O-alkylène en (C₁-C₆)-NH-alkylène en (C₁-C₆)-(R9), -O-alkylène en (C₁-C₆)-N(alkylène en (C₁-C₆)-R9)₂, -NH-alkylène en (C₁-C₆)-NH₂, -NH-alkylène en (C₁-C₆)-NH-alkylène en (C₁-C₆)-(R9), -NH-alkylène en (C₁-C₆)-N-(alkylène en (C₁-C₆)-R9)₂, SO₂-CH₃, SO₂-NH₂, SO₂-NH-alkyle en (C₁-C₆), SO₂-N(alkyle en (C₁-C₆))₂, COOH, COO-alkyle en (C₁-C₆), CONH₂, CONH-alkyle en (C₁-C₆), CON(alkyle en (C₁-C₆)₂, SF₅ ;
aryle en (C₆-C₁₀), alkylène en (C₁-C₆)-aryle en (C₆-C₁₀),
le radical aryle pouvant être substitué une à 3 fois avec F, Cl, Br, I, OH, CF₃, CHF₂, CH₂F, NO₂, CN, OCF₃, OCHF₂, O-alkyle en (C₁-C₆), alkyle en (C₁-C₆), NH₂, NH-alkyle en (C₁-C₆), N(alkyle en (C₁-C₆))₂, SO₂-CH₃, SO₂-NH₂, SO₂-NH-alkyle en (C₁-C₆), SO₂-N(alkyle en (C₁-C₆))₂, COOH, COO-alkyle en (C₁-C₆), CONH₂, CONH-alkyle en (C₁-C₆), CON(alkyle en (C₁-C₆))₂, SF₅, aryle en (C₆-C₁₀), cycloalkyle en (C₃-C₇), hétérocycle de 4 à 12 éléments ;
le radical aryle en (C₆-C₁₀), le radical cycloalkyle en (C₃-C₇), l'hétérocycle de 4 à 12 éléments pouvant être substitués une à 3 fois avec F, Cl, Br, I, OH, CF₃, CHF₂, CH₂F, NO₂, CN, OCF₃, OCHF₂, O-alkyle en (C₁-C₆), alkyle en (C₁-C₆), NH₂, NH-alkyle en (C₁-C₆), N(alkyle en (C₁-C₆))₂, SO₂-CH₃, SO₂-NH₂, SO₂-NH-alkyle en (C₁-C₆), SO₂-N(alkyle en (C₁-C₆))₂, COOH, COO-alkyle en (C₁-C₆), CONH₂, CONH-alkyle en (C₁-C₆), CON(alkyle en (C₁-C₆))₂, SF₅ ;
hétérocycle de 4 à 12 éléments, alkylène en (C₁-C₆)-hétérocycle de 4 à 12 éléments,
le radical hétérocyclyle pouvant être substitué une à 3 fois avec F, Cl, Br, I, OH, CF₃, CHF₂, CH₂F, NO₂, CN, OCF₃, OCHF₂, O-alkylène en (C₁-C₆)-(R9), alkylène en (C₁-C₆)-(R9), NH₂, NH-alkylène en (C₁-C₆)-(R9), N(alkyle en (C₁-C₆))₂, SO₂-alkylène en (C₁-C₆)-(R9), SO₂-C₂H₂F₃, SO₂-CH₃, SO₂-NH₂, SO₂-NH-alkylène en (C₁-C₆)-(R9), SO₂-N(alkyle en (C₁-C₆))₂, COOH, COO-alkylène en (C₁-C₆)-(R9), CONH₂, CONH-alkylène en (C₁-C₆)-(R9), CON(alkyle en (C₁-C₆))₂, SF₅, aryle en (C₆-C₁₀), cycloalkyle en (C₃-C₇), hétérocycle de 4 à 12 éléments ;
le radical aryle en (C₆-C₁₀), le radical cycloalkyle en (C₃-C₇), l'hétérocycle de 4 à 12 éléments pouvant être substitués une à 3 fois avec F, Cl, Br, I, OH, CF₃, CHF₂, CH₂F, NO₂, CN, OCF₃, OCHF₂, O-alkyle en (C₁-C₆), alkyle en (C₁-C₆), NH₂, NH-alkyle en (C₁-C₆), N(alkyle en (C₁-C₆))₂, SO₂-CH₃, SO₂-NH₂, SO₂-NH-alkyle en (C₁-C₆), SO₂-N(alkyle en (C₁-C₆))₂, COOH, COO-alkyle en (C₁-C₆), CONH₂, CONH-alkyle en (C₁-C₆), CON(alkyle en (C₁-C₆))₂, SF₅ ;
R7, R8 signifient indépendamment l'un de l'autre H, alkyle en (C₁-C₃) ;
R9 signifie H, OH, OCH₃, OCF₃, CHF₂, CF₃ ;
R10 signifie H, CONH₂ ;
ainsi que leurs sels pharmaceutiquement compatibles.

3. composés de formule I selon la revendication 1 ou 2, **caractérisés en ce que**
R1 signifie 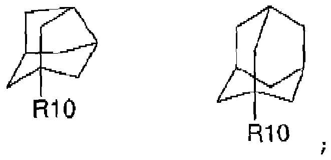
R2 signifie H ;
R3, R4, R5, R13 signifient indépendamment les uns des autres
H, F, Cl, Br, I, OH, CF₃, NO₂, CN, OCF₃, OCHF₂, alkylène en (C₁-C₆), -O-alkylène en (C₁-C₆) ;
aryle en (C₆-C₁₀),
le radical aryle pouvant être substitué une à 3 fois avec F, Cl, Br, I, OH, CF₃, CHF₂, CH₂F, NO₂, CN, OCF₃, OCHF₂, O-alkyle en (C₁-C₆), alkyle en (C₁-C₆), NH₂, NH-alkyle en (C₁-C₆), N(alkyle en (C₁-C₆))₂, SO₂-CH₃, SO₂-NH₂, SO₂-NH-alkyle en (C₁-C₆), SO₂-N(alkyle en (C₁-C₆))₂, COOH, COO-alkyle en (C₁-C₆), CONH₂, CONH-alkyle en (C₁-C₆), CON(alkyle en (C₁-C₆))₂, SF₅ ;
hétérocycle de 4 à 12 éléments,
le radical hétérocyclyle pouvant être substitué une à 3 fois avec F, Cl, Br, I, OH, CF₃, CHF₂, CH₂F, NO₂, CN, OCF₃, OCHF₂, O-alkylène en (C₁-C₆)-(R9), alkylène en (C₁-C₆)-(R9), NH₂, NH-alkylène en (C₁-C₆)-(R9), N(alkyle en (C₁-C₆))₂, SO₂-alkylène en (C₁-C₆)-(R9), SO₂-C₂H₂F₃, SO₂-CH₃, SO₂-NH₂, SO₂-NH-alkylène en (C₁-C₆)-(R9), SO₂-N(alkyle en (C₁-C₆))₂, COOH, COO-alkylène en (C₁-C₆)-(R9), CONH₂, CONH-alkylène en (C₁-C₆)-(R9), CON(alkyle en (C₁-C₆))₂, SF₅ ;
R7, R8 signifient H, alkyle en (C₁-C₃) ;
R9 signifie H, OH, OCH₃, OCF₃, CHF₂, CF₃ ;
R10 signifie H, CONH₂ ;
ainsi que leurs sels pharmaceutiquement compatibles.

4. Composés de formule I selon une ou plusieurs des revendications 1 à 3, **caractérisés en ce que** R1 signifie 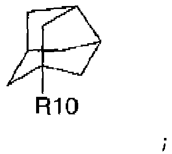
R2 signifie H ;
R3, R4, R5 signifient indépendamment les uns des autres
H, F, Cl, Br, I, OH, CF₃, NO₂, CN, OCF₃, OCHF₂, alkylène en (C₁-C₆), -O-alkylène en (C₁-C₆)
phényle,
le radical phényle pouvant être substitué une à 3 fois avec F, Cl, Br, I, OH, CF₃, CHF₂, CH₂F, NO₂, CN, OCF₃, OCHF₂, O-alkyle en (C₁-C₆), alkyle en (C₁-C₆), NH₂, NH-alkyle en (C₁-C₆), N(alkyle en (C₁-C₆))₂, SO₂-CH₃, SO₂-NH₂, SO₂-NH-alkyle en (C₁-C₆), SO₂-N(alkyle en (C₁-C₆))₂, COOH, COO-alkyle en (C₁-C₆), CONH₂, CONH-alkyle en (C₁-C₆), CON(alkyle en (C₁-C₆))₂, SF₅ ;
pipéridinyle,
le radical pipéridinyle pouvant être substitué une à 3 fois avec F, Cl, Br, I, OH, CF₃, CHF₂, CH₂F, NO₂, CN, OCF₃, OCHF₂, O-alkylène en (C₁-C₆)-(R9), alkylène en (C₁-C₆)-(R9), NH₂, NH-alkylène en (C₁-C₆)-(R9), N(alkyle en (C₁-C₆))₂, SO₂-alkylène en (C₁-C₆)-(R9), SO₂-C₂H₂F₃, SO₂-CH₃, SO₂-NH₂, SO₂-NH-alkylène en (C₁-C₆)-(R9), SO₂-N(alkyle en (C₁-C₆))₂, COOH, COO-alkylène en (C₁-C₆)-(R9), CONH₂, CONH-alkylène en (C₁-C₆)-(R9), CON(alkyle en (C₁-C₆))₂, SF₅ ;
R13 signifie F, Cl, Br, I, OH, CF₃, NO₂, CN, OCF₃, OCHF₂, alkylène en (C₁-C₆), -O-alkylène en (C₁-C₆) ;
phényle,
le radical phényle pouvant être substitué une à 3 fois avec F, Cl, Br, I, OH, CF₃, CHF₂, CH₂F, NO₂, CN, OCF₃, OCHF₂, O-alkyle en (C₁-C₆), alkyle en (C₁-C₆), NH₂, NH-alkyle en (C₁-C₆), N(alkyle en (C₁-C₆))₂, SO₂-CH₃, SO₂-NH₂, SO₂-NH-alkyle en (C₁-C₆), SO₂-N(alkyle en (C₁-C₆))₂, COOH, COO-alkyle en (C₁-C₆), CONH₂, CONH-alkyle en (C₁-C₆), CON(alkyle en (C₁-C₆))₂, SF₅ ;
pipéridinyle,
le radical pipéridinyle pouvant être substitué une à 3 fois avec F, Cl, Br, I, OH, CF₃, CHF₂, CH₂F, NO₂, CN, OCF₃, OCHF₂, O-alkylène en (C₁-C₆)-(R9), alkylène en (C₁-C₆)-(R9), NH₂, NH-alkylène en (C₁-C₆)-(R9), N(alkyle en (C₁-C₆))₂, SO₂-alkylène en (C₁-C₆)-(R9), SO₂-C₂H₂F₃, SO₂-CH₃, SO₂-NH₂, SO₂-NH-alkylène en (C₁-C₆)-(R9), SO₂-N(alkyle en (C₁-C₆))₂, COOH, COO-alkylène en (C₁-C₆)-(R9), CONH₂, CONH-alkylène en (C₁-C₆)-(R9), CON(alkyle en (C₁-C₆))₂, SF₅ ;
R7, R8 signifient H, alkyle en (C₁-C₃) ;
R9 signifie H, OH, OCH₃, OCF₃, CHF₂, CF₃ ;
R10 signifie H, CONH₂ ;
ainsi que leurs sels pharmaceutiquement compatibles.

5. Composés de formule I selon une ou plusieurs des revendications 1 à 4, **caractérisés en ce que** le radical R1 est relié comme illustré dans la formule Ia et Ib 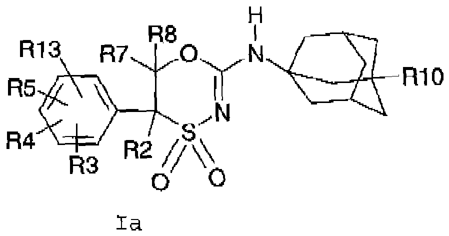 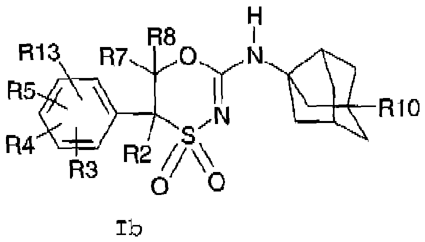 ainsi que leurs sels pharmaceutiquement compatibles.

6. Composés de formule I selon une ou plusieurs des revendications 1 à 5, pour une utilisation en tant que médicament.

7. Médicament contenant des composés de formule I selon une ou plusieurs des revendications 1 à 5.

8. Médicament contenant des composés de formule I selon une ou plusieurs des revendications 1 à 5 et au moins un agent actif supplémentaire.

9. Médicament selon la revendication 8, **caractérisé en ce qu'**il contient en tant qu'agent actif supplémentaire un ou plusieurs antidiabétiques, agents actifs hypoglycémiques, inhibiteurs d'HMGCoA-réductase, inhibiteurs de la résorption de cholestérol, agonistes de PPAR gamma, agonistes de PPAR alpha, agonistes de PPAR alpha/gamma, agonistes de PPAR delta, fibrates, inhibiteurs de MTP, inhibiteurs de la résorption d'acide biliaire, inhibiteurs de CETP, adsorbeurs polymères d'acide biliaire, inducteurs des récepteurs de LDL, inhibiteurs d'ACAT, antioxydants, inhibiteurs de lipoprotéine lipase, inhibiteurs d'ATP citrate lyase, inhibiteurs de squalène synthétase, antagonistes de lipoprotéine (a), agonistes du récepteur HM74A, inhibiteurs de lipase, insulines, sulfonylurées, biguanides, méglitinides, thiazolidinediones, inhibiteurs d'α-glucosidase, agents actifs agissant sur le canal potassique ATP-dépendant des cellules bêta, inhibiteurs de glycogène phosphorylase, antagonistes des récepteurs de glucagon, activateurs de glucokinase, inhibiteurs de gluconéogenèse, inhibiteurs de fructose 1,6-biphosphatase, modulateurs du transporteur de glucose 4, inhibiteurs de glutamine fructose 6-phosphate amidotransférase, inhibiteurs de dipeptidylpeptidase IV, inhibiteurs de 11-bêta-hydroxystéroïde déshydrogénasse 1, inhibiteurs de protéine tyrosine phosphatase 1B, modulateurs du transporteur de glucose 1 ou 2 dépendant du sodium, modulateurs de GPR40, inhibiteurs de la lipase hormonosensible, inhibiteurs d'acétyl-CoA carboxylase, inhibiteurs de phosphoénolpyruvate-carboxykinase, inhibiteurs de glycogène synthase kinase-3 bêta, inhibiteurs de protéine kinase C bêta, antagonistes du récepteur d'endothéline A, inhibiteurs de I kappaB kinase, modulateurs du récepteur de glucocorticoïdes, agonistes de CART, agonistes de NPY, agonistes de MC4, agonistes d'orexine, agonistes d'H3, agonistes de TNF, antagonistes de CRF, antagonistes de CRF BP, agonistes d'urocortine, agonistes de β3, antagonistes du récepteur de CB1, agonistes de MSH (hormone mélanotrope), agonistes de CCK, inhibiteurs du recaptage de la sérotonine, composés mixtes sérotoninergiques et noradrénergiques, agonistes de 5HT, agonistes de bombésine, antagonistes de galanine, hormones de croissance, composés libérant des hormones de croissance, agonistes de TRH, modulateurs 2 ou 3 des protéines découplantes, agonistes de leptine, agonistes de DA, inhibiteurs de lipase/amylase, modulateurs de PPAR, modulateurs de RXR ou agonistes de TR-β ou amphétamines.

10. Procédé de fabrication d'un médicament contenant les composés de formule I selon une ou plusieurs des revendications 1 à 5, **caractérisé en ce que** l'agent actif est mélangé avec un support pharmaceutiquement approprié et ce mélange est mis sous une forme appropriée pour l'administration.

11. Utilisation des composés de formule I selon une ou plusieurs des revendications 1 à 5 pour la fabrication d'un médicament pour le traitement de l'hyperglycémie.

12. Utilisation des composés de formule I selon une ou plusieurs des revendications 1 à 5 pour la fabrication d'un médicament pour le traitement du diabète.

13. Utilisation des composés de formule I selon une ou plusieurs des revendications 1 à 5 pour la fabrication d'un médicament pour le traitement de la résistance à l'insuline.

14. Ensemble (kit) constitué d'emballages séparés de
a) une quantité efficace d'un composé de formule I selon une ou plusieurs des revendications 1 à 5 et
b) une quantité efficace d'un agent actif médicamenteux supplémentaire.
